# EUROPEAN PATENT SPECIFICATION

(11) **EP 2 099 759 B1**
(45) Date of publication and mention of the grant of the patent: **18.05.2011**
(21) Application number: 07847814.6
(22) Date of filing: 04.12.2007
(51) Int. Cl.: C07D 215/14, C07D 401/04, C07D 413/04, C07D 417/04, A61K 31/496, A61P 31/04

(54) **ANTIBACTERIAL QUINOLINE DERIVATIVES**
ANTIBAKTERIELLE CHINOLINDERIVATE
DÉRIVÉS DE QUINOLINE ANTIBACTÉRIENS

(30) Priority: 06.12.2006 EP 06125529
(43) Date of publication of application: 16.09.2009
(73) Proprietor: Janssen Pharmaceutica NV, 2340 Beerse (BE)
(72) Inventor: GUILLEMONT, Jérôme Emile Georges, 27106 Val de Reuil Cedex (FR); ANDRIES, Koenraad Jozef Lodewijk Marcel, 2340 Beerse (BE); KOUL, Anil, 2340 Beerse (BE)
(74) Representative: Vervoort, Liesbeth
(86) International application number: PCT/EP2007/063316
(87) International publication number: WO 2008/068270

(56) References cited:
- WO-A-2004/011436
- WO-A-2005/117875

## Description

The present invention relates to novel substituted quinoline derivatives useful for the treatment of bacterial diseases, including but not limited to diseases caused by pathogenic mycobacteria such as *Mycobacterium tuberculosis, M. bovis, M. leprae, M. avium and M. marinum,* or pathogenic Staphylococci or Streptococci.

### BACKGROUND OF THE INVENTION

*Mycobacterium tuberculosis* is the causative agent of tuberculosis (TB), a serious and potentially fatal infection with a world-wide distribution. Estimates from the World Health Organization indicate that more than 8 million people contract TB each year, and 2 million people die from tuberculosis yearly. In the last decade, TB cases have grown 20% worldwide with the highest burden in the most impoverished communities. If these trends continue, TB incidence will increase by 41 % in the next twenty years. Fifty years since the introduction of an effective chemotherapy, TB remains after AIDS, the leading infectious cause of adult mortality in the world. Complicating the TB epidemic is the rising tide of multi-drug- resistant strains, and the deadly symbiosis with HIV. People who are HIV-positive and infected with TB are 30 times more likely to develop active TB than people who are HIV-negative and TB is responsible for the death of one out of every three people with HIV/AIDS worldwide

Existing approaches to treatment of tuberculosis all involve the combination of multiple agents. For example, the regimen recommended by the U.S. Public Health Service is a combination of isoniazid, rifampicin and pyrazinamide for two months, followed by isoniazid and rifampicin alone for a further four months. These drugs are continued for a further seven months in patients infected with HIV. For patients infected with multi-drug resistant strains of *M. tuberculosis,* agents such as ethambutol, streptomycin, kanamycin, amikacin, capreomycin, ethionamide, cycloserine, ciprofoxacin and ofloxacin are added to the combination therapies. There exists no single agent that is effective in the clinical treatment of tuberculosis, nor any combination of agents that offers the possibility of therapy of less than six months' duration.

There is a high medical need for new drugs that improve current treatment by enabling regimens that facilitate patient and provider compliance. Shorter regimens and those that require less supervision are the best way to achieve this. Most of the benefit from treatment comes in the first 2 months, during the intensive, or bactericidal, phase when four drugs are given together; the bacterial burden is greatly reduced, and patients become noninfectious. The 4- to 6-month continuation, or sterilizing, phase is required to eliminate persisting bacilli and to minimize the risk of relapse. A potent sterilizing drug that shortens treatment to 2 months or less would be extremely beneficial. Drugs that facilitate compliance by requiring less intensive supervision also are needed. Obviously, a compound that reduces both the total length of treatment and the frequency of drug administration would provide the greatest benefit.

Complicating the TB epidemic is the increasing incidence of multi-drug- resistant strains or MDR-TB. Up to four percent of all cases worldwide are considered MDR-TB - those resistant to the most effective drugs of the four-drug standard, isoniazid and rifampin. MDR-TB is lethal when untreated and cannot be adequately treated through the standard therapy, so treatment requires up to 2 years of "second-line" drugs. These drugs are often toxic, expensive and marginally effective. In the absence of an effective therapy, infectious MDR-TB patients continue to spread the disease, producing new infections with MDR-TB strains. There is a high medical need for a new drug with a new mechanism of action, which is likely to demonstrate activity against drug resistant, in particular MDR strains.

The term "drug resistant" as used hereinbefore or hereinafter is a term well understood by the person skilled in microbiology. A drug resistant Mycobacterium is a Mycobacterium which is no longer susceptible to at least one previously effective drug; which has developed the ability to withstand antibiotic attack by at least one previously effective drug. A drug resistant strain may relay that ability to withstand to its progeny. Said resistance may be due to random genetic mutations in the bacterial cell that alters its sensitivity to a single drug or to different drugs.
MDR tuberculosis is a specific form of drug resistant tuberculosis due to a bacterium resistant to at least isoniazid and rifampicin (with or without resistance to other drugs), which are at present the two most powerful anti-TB drugs. Thus, whenever used hereinbefore or hereinafter "drug resistant" includes multi drug resistant.

Another factor in the control of the TB epidemic is the problem of latent TB. In spite of decades of tuberculosis (TB) control programs, about 2 billion people are infected by M. tuberculosis, though asymptomatically. About 10% of these individuals are at risk of developing active TB during their lifespan. The global epidemic of TB is fuelled by infection of HIV patients with TB and rise of multi-drug resistant TB strains (MDR-TB). The reactivation of latent TB is a high risk factor for disease development and accounts for 32% deaths in HIV infected individuals. To control TB epidemic, the need is to discover new drugs that can kill dormant or latent bacilli. The dormant TB can get reactivated to cause disease by several factors like suppression of host immunity by use of immunosuppressive agents like antibodies against tumor necrosis factor α or interferon-γ. In case of HIV positive patients the only prophylactic treatment available for latent TB is two- three months regimens of rifampicin, pyrazinamide. The efficacy of the treatment regime is still not clear and furthermore the length of the treatments is an important constrain in resource-limited environments. Hence there is a drastic need to identify new drugs, which can act as chemoprophylatic agents for individuals harboring latent TB bacilli.
The tubercle bacilli enter healthy individuals by inhalation; they are phagocytosed by the alveolar macrophages of the lungs. This leads to potent immune response and formation of granulomas, which consist of macrophages infected with M. tuberculosis surrounded by T cells. After a,period of 6-8 weeks the host immune response cause death of infected cells by necrosis and accumulation of caseous material with certain extracellular bacilli, surrounded by macrophages, epitheloid cells and layers of lymphoid tissue at the periphery. In case of healthy individuals, most of the mycobacteria are killed in these environments but a small proportion of bacilli still survive and are thought to exist in a non-replicating, hypometabolic state and are tolerant to killing by anti-TB drugs like isoniazid. These bacilli can remain in the altered physiological environments even for individual's lifetime without showing any clinical symptoms of disease. However, in 10% of the cases these latent bacilli may reactivate to cause disease. One of the hypothesis about development of these persistent bacteria is patho-physiological environment in human lesions namely, reduced oxygen tension, nutrient limitation, and acidic pH. These factors have been postulated to render these bacteria phenotypically tolerant to major anti-mycobacterial drugs.

In addition to the management of the TB epidemic, there is the emerging problem of resistance to first-line antibiotic agents. Some important examples include penicillin-resistant Streptococcus pneumoniae, vancomycin-resistant enterococci, methicillin-resistant Staphylococcus aureus, multi-resistant salmonellae.

The consequences of resistance to antibiotic agents are severe. Infections caused by resistant microbes fail to respond to treatment, resulting in prolonged illness and greater risk of death. Treatment failures also lead to longer periods of infectivity, which increase the numbers of infected people moving in the community and thus exposing the general population to the risk of contracting a resistant strain infection.
Hospitals are a critical component of the antimicrobial resistance problem worldwide. The combination of highly susceptible patients, intensive and prolonged antimicrobial use, and cross-infection has resulted in infections with highly resistant bacterial pathogens.

Self-medication with antimicrobials is another major factor contributing to resistance. Self-medicated antimicrobials may be unnecessary, are often inadequately dosed, or may not contain adequate amounts of active drug.

Patient compliance with recommended treatment is another major problem. Patients forget to take medication, interrupt their treatment when they begin to feel better, or may be unable to afford a full course, thereby creating an ideal environment for microbes to adapt rather than be killed.

Because of the emerging resistance to multiple antibiotics, physicians are confronted with infections for which there is no effective therapy. The morbidity, mortality, and financial costs of such infections impose an increasing burden for health care systems worldwide.

Therefore, there is a high need for new compounds to treat bacterial infections, especially mycobacterial infections including drug resistant and latent mycobacterial infections, and also other bacterial infections especially those caused by resistant bacterial strains.

W02004/011436, WO2005/070924 WO2005/070430 and WO2005/075428 disclose certain substituted quinoline derivatives having activity against *Mycobacteria,* in particular against *Mycobacterium tuberculosis.* WO2005/117875 describes substituted quinoline derivatives having activity against resistant Mycobacterial strains. WO2006/067048 describes substituted quinoline derivatives having activity against latent tuberculosis. One particular compound of these substituted quinoline derivatives is described in Science (2005), 307, 223-227 and its mode of action is described in WO2006/035051.

Other substituted quinolines are disclosed in US-5,965,572 (The United States of America) for treating antibiotic resistant infections and in WO00/34265 to inhibit the growth of bacterial microorganisms.

The purpose of the present invention is to provide novel compounds, in particular substituted quinoline derivatives, having the property of inhibiting bacterial growth especially of Streptococci, Staphylococci or mycobacteria and therefore useful for the treatment of bacterial diseases, particularly those diseases caused by pathogenic bacteria such as *Streptococcus pneumonia, Staphylococcus aureus* or *Mycobacterium tuberculosis* (including the latent disease and including drug resistant *M. tuberculosis* strains), *M. bovis, M. leprae, M. avium and M. marinum.*

### SUMMARY OF THE INVENTION

The present invention relates to novel substituted quinoline derivatives according to formula (Ia) or (Ib): 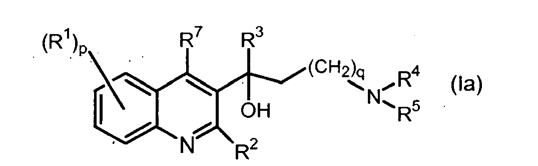 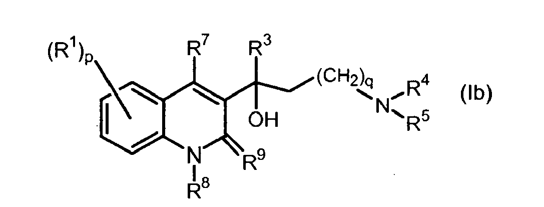 including any stereochemically isomeric form thereof, wherein
- p: is an integer equal to 1, 2, 3 or 4;
- q: is an integer equal to zero, 1, 2, 3 or 4 ;
- R¹: is hydrogen, cyano, formyl, carboxyl, halo, alkyl, C₂₋₆alkenyl, C₂-₆alkynyl, haloalkyl, hydroxy, alkyloxy, alkylthio, alkylthioalkyl, -C=N-OR¹¹, amino, mono or di(alkyl)amino, aminoalkyl, mono or di(alkyl)aminoalkyl, alkylcarbonylaminoalkyl, aminocarbonyl, mono or di(alkyl)aminocarbonyl, arylalkyl, arylcarbonyl, R^{5a}R^{4a}Nalkyl, di(aryl)alkyl, aryl, R^{5a}R^{4a}N-, R^{5a}R^{4a}N-C(=O)-, or Het;
- R²: is hydrogen, alkyloxy, aryl, aryloxy, hydroxy, mercapto, alkyloxyalkyloxy, alkylthio, mono or di(alkyl)amino, pyrrolidino or a radical of formula 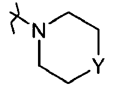 wherein Y is CH₂, O, S, NH or N-alkyl ;
- R³: is alkyl, arylalkyl, aryl-O-alkyl, aryl-alkyl-O-alkyl, aryl, aryl-aryl, Het, Het-alkyl, Het-O-alkyl, Het-alkyl-O-alkyl or 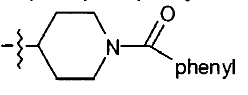
- R⁴ and R⁵: each independently is hydrogen; alkyl; alkyloxyalkyl; arylalkyl; Het-alkyl; mono- or dialkylaminoalkyl; bicyclo[2.2.1]heptyl; Het; aryl; or -C(=NH)-NH2; or
- R⁴ and R⁵: together with the nitrogen atom to which they are attached form a radical selected from the group consisting of pyrrolidino, piperidino, piperazino, morpholino, 4-thiomorpholino, 1,1-dioxide-thiomorpholinyl, azetidinyl, 2,3-dihydroisoindol-1-yl, thiazolidin-3-yl, 1,2,3,6- tetrahydropyridyl, hexahydro-1*H-*azepinyl, hexahydro-1*H-*1,4- diazepinyl, hexahydro-1,4-oxazepinyl, 1,2,3,4-tetrahydroisoquinolin-2- yl, 2,5-diazabicyclo[2.2.1]heptyl, pyrrolinyl, pyrrolyl, imidazolidinyl, pyrazolidinyl, 2-imidazolinyl, 2-pyrazolinyl, imidazolyl, pyrazolyl, triazolyl, pyridinyl, pyridazinyl, pyrimidinyl, pyrazinyl and triazinyl, each radical optionally substituted with 1, 2, 3 or 4 substituents, each substituent independently selected from alkyl, haloalkyl, alkylcarbonyl, halo, arylalkyl, hydroxy, alkyloxy, amino, mono- or dialkylamino, aminoalkyl, mono- or dialkylaminoalkyl, alkylthio, alkylthioalkyl, aryl, pyridyl, pyrimidinyl, piperidinyl optionally substituted with alkyl or pyrrolidinyl optionally substituted with arylalkyl;
- R^{4a} and R^{5a}: together with the nitrogen atom to which they are attached form a radical selected from the group consisting of pyrrolidino, piperidino, piperazino, morpholino, 4-thiomorpholino, 2,3-dihydroisoindol-1-yl, thiazolidin-3-yl, 1,2,3,6-tetrahydropyridyl, hexahydro-1*H-*azepinyl, hexahydro-1*H-*1,4-diazepinyl, hexahydro-1,4-oxazepinyl, 1,2,3,4- tetrahydroisoquinolin-2-yl, pyrrolinyl, pyrrolyl, imidazolidinyl, pyrazolidinyl, 2-imidazolinyl, 2-pyrazolinyl, imidazolyl, pyrazolyl, triazolyl, pyridinyl, pyridazinyl, pyrimidinyl, pyrazinyl and triazinyl, each radical optionally substituted with 1, 2, 3 or 4 substituents, each substituent independently selected from alkyl, haloalkyl, halo, arylalkyl, hydroxy, alkyloxy, amino, mono- or dialkylamino, alkylthio, alkylthioalkyl, aryl, pyridyl or pyrimidinyl;
- R⁷: is hydrogen, halo, alkyl, aryl or Het;
- R⁸: is hydrogen or alkyl;
- R⁹: is oxo; or
- R⁸ and R⁹: together form the radical -CH=CH-N=;
- R¹¹: is hydrogen or alkyl;
- aryl: is a homocycle selected from phenyl, naphthyl, acenaphthyl or tetrahydronaphthyl, each being optionally substituted with 1, 2 or 3 substituents, each substituent being independently selected from hydroxy, halo, cyano, nitro, amino, mono- or dialkylamino, alkyl, C₂-₆alkenyl optionally substituted with phenyl, haloalkyl, alkyloxy, haloalkyloxy, carboxyl, alkyloxycarbonyl, aminocarbonyl, morpholinyl or mono- or dialkylaminocarbonyl;
- Het: is a monocyclic heterocycle selected from *N-*phenoxypiperidinyl, piperidinyl, pyrrolyl, pyrazolyl, imidazolyl, furanyl, thienyl, oxazolyl, isoxazolyl, thiazolyl, isothiazolyl, pyridinyl, pyrimidinyl, pyrazinyl or pyridazinyl; or a bicyclic heterocycle selected from quinolinyl, quinoxalinyl, indolyl, benzimidazolyl, benzoxazolyl, benzisoxazolyl, benzothiazolyl, benzisothiazolyl, benzofuranyl, benzothienyl, 2,3-dihydrobenzo[1,4]dioxinyl or benzo[1,3]dioxolyl; each monocyclic and bicyclic heterocycle being optionally substituted with 1, 2 or 3 substituents, each substituent independently selected from halo, hydroxy, alkyl or alkyloxy;
the *N-*oxides thereof, the pharmaceutically acceptable salts thereof or the solvates thereof.

Whenever used herein, the term "compounds of formula (Ia) or (Ib)" or "compounds according to the invention" is meant to also include their pharmaceutically acceptable salts or their *N-*oxide forms or their solvates.

The compounds of formula (Ia) and (Ib) are interrelated in that e.g. a compound according to formula (Ib), with R⁹ equal to oxo and R⁸ equal to hydrogen, is the tautomeric equivalent of a compound according to formula (Ia) with R² equal to hydroxy (keto-enol tautomerism).

In the definition of Het, it is meant to include all the possible isomeric forms of the heterocycles, for instance, pyrrolyl comprises 1*H-*pyrrolyl and 2*H-*pyrrolyl.

The aryl or Het listed in the definitions of the substituents of the compounds of formula (Ia) or (Ib) (see for instance R³) as mentioned hereinbefore or hereinafter may be attached to the remainder of the molecule of formula (Ia) or (Ib) through any ring carbon or heteroatom as appropriate, if not otherwise specified. Thus, for example, when Het is imidazolyl, it may be 1-imidazolyl, 2-imidazolyl, 4-imidazolyl and the like.

Lines drawn from substituents into ring systems indicate that the bond may be attached to any of the suitable ring atoms.

The pharmaceutically acceptable salts as mentioned hereinbefore or hereinafter are meant to comprise the therapeutically active non-toxic acid addition salt forms which the compounds according to formula (Ia) or formula (Ib) are able to form. Said acid addition salts can be obtained by treating the base form of the compounds according to formula (Ia) or formula (Ib) with appropriate acids, for example inorganic acids, for example hydrohalic acid, in particular hydrochloric acid, hydrobromic acid, sulfuric acid, nitric acid and phosphoric acid; organic acids, for example acetic acid, hydroxyacetic acid, propanoic acid, lactic acid, pyruvic acid, oxalic acid, malonic acid, succinic acid, maleic acid, fumaric acid, malic acid, tartaric acid, citric acid, methanesulfonic acid, ethanesulfonic acid, benzenesulfonic acid, p-toluenesulfonic acid, cyclamic acid, salicyclic acid, p-aminosalicylic acid and pamoic acid.

The compounds of formula (Ia) or (Ib) containing acidic protons may be converted into their therapeutically active non-toxic metal or amine addition salt forms by treatment with appropriate organic and inorganic bases. The pharmaceutically acceptable salts as mentioned hereinbefore or hereinafter are meant to also comprise the therapeutically active non-toxic metal or amine addition salt forms (base addition salt forms) which the compounds of formula (Ia) or (Ib) are able to form. Appropriate base addition salt forms comprise, for example, the ammonium salts, the alkali and earth alkaline metal salts, e.g. the lithium, sodium, potassium, magnesium, calcium salts and the like, salts with organic bases, e.g. primary, secondary and tertiary aliphatic and aromatic amines such as methylamine, ethylamine, propylamine, isopropylamine, the four butylamine isomers, dimethylamine, diethylamine, diethanolamine, dipropylamine, diisopropylamine, di-*n*-butylamine, pyrrolidine, piperidine, morpholine, trimethylamine, triethylamine, tripropylamine, quinuclidine, pyridine, quinoline and isoquinoline, the benzathine, *N-*methyl-D-glucamine, 2-amino-2-(hydroxymethyl)-1,3-propanediol, hydrabamine salts, and salts with amino acids such as, for example, arginine, lysine and the like.

Conversely, said acid or base addition salt forms can be converted into the free forms by treatment with an appropriate base or acid.

The term pharmaceutically acceptable salt also comprises the quaternary ammonium salts (quaternary amines) which the compounds of formula (Ia) or (Ib) are able to form by reaction between a basic nitrogen of a compound of formula (Ia) or (Ib) and an appropriate quaternizing agent, such as, for example, an optionally substituted C₁₋₆alkylhalide, arylC₁₋₆alkylhalide, C₁₋₆alkylcarbonylhalide, arylcarbonylhalide, HetC₁₋₆alkylhalide or Hetcarbonylhalide, e.g. methyliodide or benzyliodide. Preferably, Het represents a monocyclic heterocycle selected from furanyl or thienyl; or a bicyclic heterocycle selected from benzofuranyl or benzothienyl; each monocyclic and bicyclic heterocycle may optionally be substituted with 1, 2 or 3 substituents, each substituent independently selected from the group of halo, alkyl and aryl. Preferably, the quaternizing agent is C₁₋₆alkylhalide. Other reactants with good leaving groups may also be used, such as C₁₋₆alkyl trifluoromethanesulfonates, C₁₋₆alkyl methanesulfonates, and C₁₋₆alkyl p-toluenesulfonates. A quaternary amine has a positively charged nitrogen. Pharmaceutically acceptable counterions include chloro, bromo, iodo, trifluoroacetate, acetate, triflate, sulfate, sulfonate. Preferably, the counterion is iodo. The counterion of choice can be introduced using ion exchange resins.

The term solvate comprises the hydrates and solvent addition forms which the compounds of formula (Ia) or (Ib) are able to form, as well as the salts thereof. Examples of such forms are e.g. hydrates, alcoholates and the like.

In the framework of this application, a compound according to the invention is inherently intended to comprise all stereochemically isomeric forms thereof. The term "stereochemically isomeric forms" as used hereinbefore or hereinafter defines all the possible stereoisomeric forms which the compounds of formula (Ia) and (Ib), and their *N-*oxides, pharmaceutically acceptable salts, solvates or physiologically functional derivatives may possess. Unless otherwise mentioned or indicated, the chemical designation of compounds denotes the mixture of all possible stereochemically isomeric forms.
In particular, stereogenic centers may have the R- or S-configuration; substituents on bivalent cyclic (partially) saturated radicals may have either the *cis-* or *trans-*configuration. Compounds encompassing double bonds can have an E (entgegen) or Z (zusammen) -stereochemistry at said double bond. The terms cis, trans, R, S, E and Z are well known to a person skilled in the art.
Stereochemically isomeric forms of the compounds of formula (Ia) and (Ib) are obviously intended to be embraced within the scope of this invention.
Of special interest are those compounds of formula (Ia) or (Ib) which are stereochemically pure.

Following CAS-nomenclature conventions, when two stereogenic centers of known absolute configuration are present in a molecule, an R or S descriptor is assigned (based on Cahn-Ingold-Prelog sequence rule) to the lowest-numbered chiral center, the reference center. The configuration of the second stereogenic center is indicated using relative descriptors [*R*,R** ] or [*R**,*S**], where *R** is always specified as the reference center and [*R*,R**] indicates centers with the same chirality and [*R*,S**] indicates centers of unlike chirality. For example, if the lowest-numbered chiral center in the molecule has an S configuration and the second center is *R*, the stereo descriptor would be specified as *S-*[*R*,S**]*.* If "α" and "α" are used : the position of the highest priority substituent on the asymmetric carbon atom in the ring system having the lowest ring number, is arbitrarily always in the "α" position of the mean plane determined by the ring system. The position of the highest priority substituent on the other asymmetric carbon atom in the ring system relative to the position of the highest priority substituent on the reference atom is denominated "α", if it is on the same side of the mean plane determined by the ring system, or "β", if it is on the other side of the mean plane determined by the ring system.

When a specific stereoisomeric form is indicated, this means that said form is substantially free, i.e. associated with less than 50 %, preferably less than 20 %, more preferably less than 10 %, even more preferably less than 5 %, further preferably less than 2 % and most preferably less than 1 % of the other isomer(s). Thus, when a compound of formula (Ia) or (Ib) is for instance specified as (S), this means that the compound is substantially free of the (R) isomer.

The compounds of either formula (la) and (Ib) may be synthesized in the form of mixtures, in particular racemic mixtures, of enantiomers which can be separated from one another following art-known resolution procedures. The racemic compounds of either formula (Ia) and (Ib) may be converted into the corresponding diastereomeric salt forms by reaction with a suitable chiral acid. Said diastereomeric salt forms are subsequently separated, for example, by selective or fractional crystallization and the enantiomers are liberated therefrom by alkali. An alternative manner of separating the enantiomeric forms of the compounds of either formula (Ia) and (Ib) involves liquid chromatography using a chiral stationary phase. Said pure stereochemically isomeric forms may also be derived from the corresponding pure stereochemically isomeric forms of the appropriate starting materials, provided that the reaction occurs stereospecifically. Preferably if a specific stereoisomer is desired, said compound will be synthesized by stereospecific methods of preparation. These methods will advantageously employ enantiomerically pure starting materials.

The tautomeric forms of the compounds of formula (Ia) or (Ib) are meant to comprise those compounds of formula (Ia) or (Ib) wherein e.g. an enol group is converted into a keto group (keto-enol tautomerism). Tautomeric forms of the compounds of formula (Ia) and (Ib) or of intermediates of the present invention are intended to be embraced by the ambit of this invention.

The *N-*oxide forms of the present compounds are meant to comprise the compounds of formula (Ia) or (Ib) wherein one or several tertiary nitrogen atoms are oxidized to the so-called *N-*oxide.

The compounds of formula (Ia) and (Ib) may be converted to the corresponding *N-*oxide forms following art-known procedures for converting a trivalent nitrogen into its *N-*oxide form. Said *N-*oxidation reaction may generally be carried out by reacting the starting material of formula (Ia) or (Ib) with an appropriate organic or inorganic peroxide. Appropriate inorganic peroxides comprise, for example, hydrogen peroxide, alkali metal or earth alkaline metal peroxides, e.g. sodium peroxide, potassium peroxide; appropriate organic peroxides may comprise peroxy acids such as, for example, benzenecarboperoxoic acid or halo substituted benzenecarboperoxoic acid, e.g. 3-chlorobenzenecarboperoxoic acid, peroxoalkanoic acids, e.g. peroxoacetic acid, alkylhydroperoxides, e.g. t.butyl hydro-peroxide. Suitable solvents are, for example, water, lower alcohols, e.g. ethanol and the like, hydrocarbons, e.g. toluene, ketones, e.g. 2-butanone, halogenated hydrocarbons, e.g. dichloromethane, and mixtures of such solvents.

In the framework of this application, a compound according to the invention is inherently intended to comprise all isotopic combinations of its chemical elements. In the framework of this application, a chemical element, in particular when mentioned in relation to a compound according to formula (Ia) or (Ib), comprises all isotopes and isotopic mixtures of this element, either naturally occuring or synthetically produced, either with natural abundance or in an isotopically enriched form. In particular, when hydrogen is mentioned, it is understood to refer to ¹H, ²H, ³H and mixtures thereof ; when carbon is mentioned, it is understood to refer to ¹¹C, ¹²C, ¹³C, ¹⁴C and mixtures thereof ; when nitrogen is mentioned, it is understood to refer to ¹³N, ¹⁴ N, ¹⁵N and mixtures thereof; when oxygen is mentioned, it is understood to refer to ¹⁴O, ¹⁵O, ¹⁶O, ¹⁷O, ¹⁸O and mixtures thereof ; and when fluor is mentioned, it is understood to refer to ¹⁸F, ¹⁹F and mixtures thereof.
A compound according to the invention therefore inherently comprises a compound with one or more isotopes of one or more element, and mixtures thereof, including a radioactive compound, also called radiolabelled compound, wherein one or more non-radioactive atoms has been replaced by one of its radioactive isotopes. By the term "radiolabelled compound" is meant any compound according to formula (Ia) or (Ib), a pharmaceutically acceptable salt thereof or an *N-*oxide form thereof or a solvate thereof, which contains at least one radioactive atom. For example, a compound can be labelled with positron or with gamma emitting radioactive isotopes. For radioligand-binding techniques (membrane receptor assay), the ³H-atom or the ¹²⁵I-atom is the atom of choice to be replaced. For imaging, the most commonly used positron emitting (PET) radioactive isotopes are ¹¹C, ¹⁸F, ¹⁵O and ¹³N, all of which are accelerator produced and have half-lives of 20, 100, 2 and 10 minutes respectively. Since the half-lives of these radioactive isotopes are so short, it is only feasible to use them at institutions which have an accelerator on site for their production, thus limiting their use. The most widely used of these are ¹⁸F, ^{99m}Tc, ²⁰¹Tl and ¹²³I*.* The handling of these radioactive isotopes, their production, isolation and incorporation in a molecule are known to the skilled person.
In particular, the radioactive atom is selected from the group of hydrogen, carbon, nitrogen, sulfur, oxygen and halogen. Preferably, the radioactive atom is selected from the group of hydrogen, carbon and halogen.
In particular, the radioactive isotope is selected from the group of ³H, ¹¹C, ¹⁸F, ¹²²I, ¹²³I, ¹²⁵I, ¹³¹I, ⁷⁵Br, ⁷⁶Br, ⁷⁷Br and ⁸²Br. Preferably, the radioactive isotope is selected from the group of ³H, ¹¹C and ¹⁸F.

In the framework of this application, alkyl is a straight or branched saturated hydrocarbon radical having from 1 to 6 carbon atoms ; or is a cyclic saturated hydrocarbon radical having from 3 to 6 carbon atoms ; or is a cyclic saturated hydrocarbon radical having from 3 to 6 carbon atoms attached to a straight or branched saturated hydrocarbon radical having from 1 to 6 carbon atoms ; wherein each carbon atom can be optionally substituted with cyano, hydroxy, C₁₋₆alkyloxy or oxo. Preferably alkyl is a straight or branched saturated hydrocarbon radical having from 1 to 6 carbon atoms ; or is a cyclic saturated hydrocarbon radical having from 3 to 6 carbon atoms ; wherein each carbon atom can be optionally substituted with hydroxyl or C₁₋₆alkyloxy.
Preferably, alkyl is methyl, ethyl or cyclohexylmethyl, more preferably methyl or ethyl. An interesting embodiment of alkyl in all definitions used hereinbefore or hereinafter is C₁₋₆alkyl which represents a straight or branched saturated hydrocarbon radical having from 1 to 6 carbon atoms such as for example methyl, ethyl, propyl, 2-methyl-ethyl, pentyl, hexyl and the like. A preferred subgroup of C₁₋₆alkyl is C₁₋₄alkyl which represents a straight or branched saturated hydrocarbon radical having from 1 to 4 carbon atoms such as for example methyl, ethyl, propyl, 2-methyl-ethyl and the like.

In the framework of this application C₂₋₆alkenyl is a straight or branched hydrocarbon radical having from 2 to 6 carbon atoms containing a double bond such as ethenyl, propenyl, butenyl, pentenyl, hexenyl and the like; C₂₋₆alkynyl is a straight or branched hydrocarbon radical having from 2 to 6 carbon atoms containing a triple bond such as ethynyl, propynyl, butynyl, pentynyl, hexynyl and the like; C₃₋₆cycloalkyl is a cyclic saturated hydrocarbon radical having from 3 to 6 carbon atoms and is generic to cyclopropyl, cyclobutyl, cyclopentyl, cyclohexyl

In the framework of this application, halo is a substituent selected from the group of fluoro, chloro, bromo and iodo and haloalkyl is a straight or branched saturated hydrocarbon radical having from 1 to 6 carbon atoms or a cyclic saturated hydrocarbon radical having from 3 to 6 carbon atoms or a cyclic saturated hydrocarbon radical having from 3 to 6 carbon atoms attached to a straight or branched saturated hydrocarbon radical having from 1 to 6 carbon atoms; wherein one or more carbon atoms are substituted with one or more halo atoms. Preferably, halo is bromo, fluoro or chloro; in particular chloro or bromo. Preferably, haloalkyl is polyhaloC₁₋₆alkyl which is defined as mono- or polyhalosubstituted C₁₋₆alkyl, for example, methyl with one or more fluoro atoms, for example, difluoromethyl or trifluoromethyl, 1,1-difluoro-ethyl and the like. In case more than one halo atom is attached to an alkyl or C₁₋₆alkyl group within the definition of haloalkyl or polyhaloC₁₋₆alkyl, they may be the same or different.

A first interesting embodiment relates to a compound of formula (Ia) or (Ib) 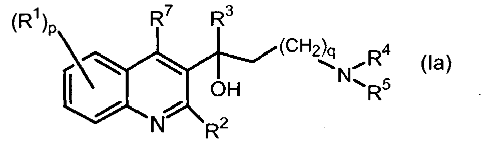 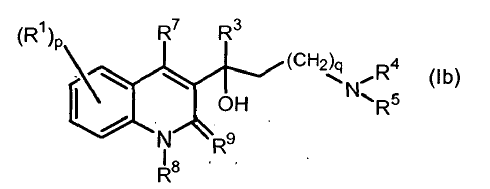 including any stereochemically isomeric form thereof, wherein
- p: is an integer equal to 1, 2, 3 or 4;
- q: is an integer equal to zero, 1, 2, 3 or 4 ;
- R¹: is hydrogen, cyano, formyl, carboxyl, halo, alkyl, C₂₋₆alkenyl, C₂₋₆alkynyl, haloalkyl, hydroxy, alkyloxy, alkylthio, alkylthioalkyl, -C=N-OR¹¹, amino, mono or di(alkyl)amino, aminoalkyl, mono or di(alkyl)aminoalkyl, alkylcarbonylaminoalkyl, aminocarbonyl, mono or di(alkyl)aminocarbonyl, arylalkyl, arylcarbonyl, R^{5a}R^{4a}Nalkyl, di(aryl)alkyl, aryl, R^{5a}R^{4a}N-, R^{5a}R^{4a}N-C(=O)-, or Het;
- R²: is hydrogen, alkyloxy, aryl, aryloxy, hydroxy, mercapto, alkyloxyalkyloxy, alkylthio, mono or di(alkyl)amino, pyrrolidino or a radical of formula 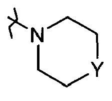 wherein Y is CH₂, O, S, NH or *N-*alkyl ;
- R³: is alkyl, arylalkyl, aryl-O-alkyl, aryl-alkyl-O-alkyl, aryl, Het, Het-alkyl, Het-O-alkyl, Het-alkyl-O-alkyl or 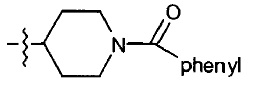
- R⁴ and R⁵: each independently is hydrogen; alkyl; alkyloxyalkyl; arylalkyl; Het-alkyl; mono- or dialkylaminoalkyl; Het; aryl; or -C(=NH)-NH₂; or
- R⁴ and R⁵: together with the nitrogen atom to which they are attached form a radical selected from the group consisting of pyrrolidino, piperidino, piperazino, morpholino, 4-thiomorpholino, 2,3-dihydroisoindol-1-yl, thiazolidin-3-yl, 1,2,3,6-tetrahydropyridyl, hexahydro-1*H-*azepinyl, hexahydro-1*H-*1,4-diazepinyl, hexahydro-1,4-oxazepinyl, 1,2,3,4- tetrahydroisoquinolin-2-yl, 2,5-diazabicyclo[2.2.1]heptyl, pyrrolinyl, pyrrolyl, imidazolidinyl, pyrazolidinyl, 2-imidazolinyl, 2-pyrazolinyl, imidazolyl, pyrazolyl, triazolyl, pyridinyl, pyridazinyl, pyrimidinyl, pyrazinyl and triazinyl, each radical optionally substituted with 1, 2, 3 or 4 substituents, each substituent independently selected from alkyl, haloalkyl, alkylcarbonyl, halo, arylalkyl, hydroxy, alkyloxy, amino, mono- or dialkylamino, alkylthio, alkylthioalkyl, aryl, pyridyl, pyrimidinyl, piperidinyl or pyrrolidinyl optionally substituted with arylalkyl;
- R^{4a} and R^{5a}: together with the nitrogen atom to which they are attached form a radical selected from the group consisting of pyrrolidino, piperidino, piperazino, morpholino, 4-thiomorpholino, 2,3-dihydroisoindol-1-yl, thiazolidin-3-yl, 1,2,3,6-tetrahydropyridyl, hexahydro-1*H-*azepinyl, hexahydro-1*H-*1,4-diazepinyl, hexahydro-1,4-oxazepinyl, 1,2,3,4- tetrahydroisoquinolin-2-yl, pyrrolinyl, pyrrolyl, imidazolidinyl, pyrazolidinyl, 2-imidazolinyl, 2-pyrazolinyl, imidazolyl, pyrazolyl, triazolyl, pyridinyl, pyridazinyl, pyrimidinyl, pyrazinyl and triazinyl, each radical optionally substituted with 1, 2, 3 or 4 substituents, each substituent independently selected from alkyl, haloalkyl, halo, arylalkyl, hydroxy, alkyloxy, amino, mono- or dialkylamino, alkylthio, alkylthioalkyl, aryl, pyridyl or pyrimidinyl;
- R⁷: is hydrogen, halo, alkyl, aryl or Het;
- R⁸: is hydrogen or alkyl;
- R⁹: is oxo; or
- R⁸ and R⁹: together form the radical -CH=CH-N=;
- R¹¹: is hydrogen or alkyl;
- aryl: is a homocycle selected from phenyl, naphthyl, acenaphthyl or tetrahydronaphthyl, each being optionally substituted with 1, 2 or 3 substituents, each substituent being independently selected from hydroxy, halo, cyano, nitro, amino, mono- or dialkylamino, alkyl, haloalkyl, alkyloxy, haloalkyloxy, carboxyl, alkyloxycarbonyl, aminocarbonyl, morpholinyl or mono- or dialkylaminocarbonyl;
- Het: is a monocyclic heterocycle selected from *N-*phenoxypiperidinyl, piperidinyl, pyrrolyl, pyrazolyl, imidazolyl, furanyl, thienyl, oxazolyl, isoxazolyl, thiazolyl, isothiazolyl, pyridinyl, pyrimidinyl, pyrazinyl or pyridazinyl; or a bicyclic heterocycle selected from quinolinyl, quinoxalinyl, indolyl, benzimidazolyl, benzoxazolyl, benzisoxazolyl, benzothiazolyl, benzisothiazolyl, benzofuranyl, benzothienyl, 2,3-dihydrobenzo[1,4]dioxinyl or benzo[1,3]dioxolyl; each monocyclic and bicyclic heterocycle being optionally substituted with 1, 2 or 3 substituents, each substituent independently selected from halo, hydroxy, alkyl or alkyloxy;
a pharmaceutically acceptable salt thereof, a *N-*oxide form thereof or a solvate thereof.

A second interesting embodiment relates to a compound of formula (Ia) or (Ib) wherein
- p: is an integer equal to 1, 2, 3 or 4;
- q: is an integer equal to zero, 1, 2, 3 or 4 ;
- R¹: is hydrogen, cyano, formyl, carboxyl, halo, C₁₋₆alkyl, C₂-₆alkenyl, C₂-₆alkynyl, polyhaloC₁₋₆alkyl, hydroxy, C₁₋₆alkyloxy, C₁₋₆alkylthio, C₁₋₆alkyloxyC₁₋₆alkyl, C₁₋₆alkylthioC₁₋₆alkyl, hydroxyC₁₋₆alkyl, -C=N-OR¹¹, amino, mono or di(C₁₋₆alkyl)amino, aminoC₁₋₆alkyl, mono or di(C₁₋₆alkyl)aminoC₁₋₆alkyl, C₁₋₆alkylcarbonylaminoC₁₋₆alkyl, aminocarbonyl, mono or di(C₁₋₆alkyl)aminocarbonyl, arylC₁₋₆a[kyl, arylcarbonyl, R^{5a}R^{4a}NC₁₋₆alkyl, di(aryl)C₁₋₆alkyl, aryl, R^{5a}R^{4a}N-, R^{5a}R^{4a}N-C(=O)-, or Het;
- R²: is hydrogen, C₁₋₆alkyloxy, aryl, aryloxy, hydroxy, mercapto, C₁₋₆alkyloxyC₁₋₆alkyloxy, C₁₋₆alkylthio, mono or di(C₁₋₆alkyl)amino, pyrrolidino or a radical of formula 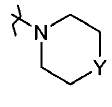 wherein Y is CH₂, O, S, NH or *N-*C₁₋₆alkyl ;
- R³: is C₁₋₆alkyl, C₃₋₆cycloalkyl, arylC₁₋₆alkyl, aryl-O-C₁₋₆alkyl, arylC₁₋₆alkyl-O-C₁₋₆alkyl, aryl, aryl-aryl, Het, Het-C₁₋₆alkyl, Het-O-C₁₋₆alkyl or HetC₁₋₆alkyl-O-C₁₋₆alkyl, or 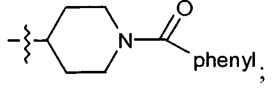
- R⁴ and R⁵: each independently is hydrogen; C₁₋₆alkyl; C₁₋₆alkyloxyC₁₋₆alkyl; arylC₁₋₆alkyl; Het-C₁₋₆alkyl; mono- or diC₁₋₆alkylaminoC₁₋₆alkyl; bicyclo[2.2.1]heptyl; Het; aryl; or -C(=NH)-NH₂; or
- R⁴ and R⁵: together with the nitrogen atom to which they are attached form a radical selected from the group consisting of pyrrolidino, piperidino, piperazino, morpholino, 4-thiomorpholino, 1,1-dioxide-thiomorpholinyl, azetidinyl, 2,3-dihydroisoindol-1-yl, thiazolidin-3-yl, 1,2,3,6- tetrahydropyridyl, hexahydro-1*H-*azepinyl, hexahydro-1*H-*1,4- diazepinyl, hexahydro-1,4-oxazepinyl, 1,2,3,4-tetrahydroisoquinolin-2- yl, 2,5-diazabicyclo[2.2.1]heptyl, pyrrolinyl, pyrrolyl, imidazolidinyl, pyrazolidinyl, 2-imidazolinyl, 2-pyrazolinyl, imidazolyl, pyrazolyl, triazolyl, pyridinyl, pyridazinyl, pyrimidinyl, pyrazinyl and triazinyl, each radical optionally substituted with 1, 2, 3 or 4 substituents, each substituent independently selected from C₁₋₆alkyl, haloC₁₋₆alkyl, C₁₋₆alkylcarbonyl, halo, arylC₁₋₆alkyl, hydroxy, C₁₋₆alkyloxy, amino, mono- or diC₁₋₆alkylamino, aminoC₁₋₆alkyl, mono- or diC₁₋₆alkylaminoC₁₋₆alkyl, C₁₋₆alkylthio, C₁₋₆alkylthioC₁₋₆alkyl, aryl, pyridyl, pyrimidinyl, piperidinyl optionally substituted with C₁₋₆alkyl or pyrrolidinyl optionally substituted with arylC₁₋₆alkyl;
- R^{4a} and R^{5a}: together with the nitrogen atom to which they are attached form a radical selected from the group consisting of pyrrolidino, piperidino, piperazino, morpholino, 4-thiomorpholino, 2,3-dihydroisoindol-1-yl, thiazolidin-3-yl, 1,2,3,6-tetrahydropyridyl, hexahydro-1*H-*azepinyl, hexahydro-1*H-*1,4-diazepinyl, hexahydro-1,4-oxazepinyl, 1,2,3,4- tetrahydroisoquinolin-2-yl, pyrrolinyl, pyrrolyl, imidazolidinyl, pyrazolidinyl, 2-imidazolinyl, 2-pyrazolinyl, imidazolyl, pyrazolyl, triazolyl, pyridinyl, pyridazinyl, pyrimidinyl, pyrazinyl and triazinyl, each radical optionally substituted with 1, 2, 3 or 4 substituents, each substituent independently selected from C₁₋₆alkyl, polyhaloC₁₋₆alkyl, halo, arylC₁₋₆alkyl, hydroxy, C₁₋₆alkyloxy, C₁₋₆alkyloxyC₁₋₆alkyl, amino, mono- or di(C₁₋₆alkyl)amino, C₁₋₆alkylthio, C₁₋₆alkyloxyC₁₋₆alkyl, C₁₋₆alkylthioC₁₋₆alkyl, aryl, pyridyl or pyrimidinyl;
- R⁷: is hydrogen, halo, C₁₋₆alkyl, aryl or Het;
- R⁸: is hydrogen or C₁₋₆alkyl;
- R⁹: is oxo; or
- R⁸ and R⁹: together form the radical -CH=CH-N=;
- R¹¹: is hydrogen or alkyl;
- aryl: is a homocycle selected from phenyl, naphthyl, acenaphthyl or tetrahydronaphthyl, each being optionally substituted with 1, 2 or 3 substituents, each substituent being independently selected from hydroxy, halo, cyano, nitro, amino, mono- or di(C₁₋₆alkyl)amino, C₁₋₆alkyl, C₂₋₆alkenyl optionally substituted with phenyl, polyhaloC₁₋₆alkyl, C₁₋₆alkyloxy, haloC₁₋₆alkyloxy, carboxyl, C₁₋₆alkyloxycarbonyl, aminocarbonyl, morpholinyl or mono- or di(C₁₋₆alkyl)aminocarbonyl;
- Het: is a monocyclic heterocycle selected from *N-*phenoxypiperidinyl, piperidinyl, pyrrolyl, pyrazolyl, imidazolyl, furanyl, thienyl, oxazolyl, isoxazolyl, thiazolyl, isothiazolyl, pyridinyl, pyrimidinyl, pyrazinyl or pyridazinyl; or a bicyclic heterocycle selected from quinolinyl, quinoxalinyl, indolyl, benzimidazolyl, benzoxazolyl, benzisoxazolyl, benzothiazolyl, benzisothiazolyl, benzofuranyl, benzothienyl, 2,3-dihydrobenzo[1,4]dioxinyl or benzo[1,3]dioxolyl; each monocyclic and bicyclic heterocycle being optionally substituted with 1, 2 or 3 substituents, each substituent independently selected from halo, hydroxy, C₁₋₆alkyl or C₁₋₆alkyloxy.

A third interesting embodiment relates to a compound of formula (Ia) or (Ib) wherein
- p: is an integer equal to 1, 2, 3 or 4;
- q: is an integer equal to zero, 1, 2, 3 or 4 ;
- R¹: is hydrogen, cyano, formyl, carboxyl, halo, C₁₋₆alkyl, C₂₋₆alkenyl, C₂₋₆alkynyl, haloC₁₋₆alkyl, hydroxy, C₁₋₆alkyloxy, C₁₋₆alkylthio, C₁₋₆alkylthio C₁₋₆alkyl, -C=N-OR¹¹, amino, mono or di(C₁₋₆alkyl)amino, amino C₁₋₆alkyl, mono or di(C₁₋₆alkyl)aminoC₁₋₆alkyl, C₁₋₆alkylcarbonylaminoC₁₋₆alkyl, aminocarbonyl, mono or di(C₁₋₆alkyl)aminocarbonyl, arylC₁₋₆alkyl, arylcarbonyl, R^{5a}R^{4a}NC₁₋₆alkyl, di(aryl)C₁₋₆alkyl, aryl, R^{5a}R^{4a}N-, R^{5a}R^{4a}N-C(=O)-, or Het;
- R²: is hydrogen, C₁₋₆alkyloxy, aryl, aryloxy, hydroxy, mercapto, C₁₋₆alkyloxyC₁₋₆alkyloxy, C₁₋₆alkylthio, mono or di(C₁₋₆alkyl)amino, pyrrolidino or a radical of formula 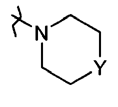 wherein Y is CH₂, O, S,
- R³: NH or *N-*C₁₋₆alkyl; is C₁₋₆alkyl, arylC₁₋₆alkyl, aryl-O-C₁₋₆alkyl, aryl-C₁₋₆alkyl-O-C₁₋₆alkyl, aryl, Het, Het-C₁₋₆alkyl, Het-O-C₁₋₆alkyl, Het-C₁₋₆alkyl-O-C₁₋₆alkyl or 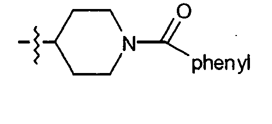
- R⁴ and R⁵: each independently is hydrogen; C₁₋₆alkyl; C₁₋₆alkyloxyC₁₋₆alkyl; arylC₁₋₆alkyl; Het-C₁₋₆alkyl; mono- or diC₁₋₆alkylaminoC₁₋₆alkyl; Het; aryl; or -C(=NH)-NH₂; or
- R⁴ and R⁵: together with the nitrogen atom to which they are attached form a radical selected from the group consisting of pyrrolidino, piperidino, piperazino, morpholino, 4-thiomorpholino, 2,3-dihydroisoindol-1-yl, thiazolidin-3-yl, 1,2,3,6-tetrahydropyridyl, hexahydro-1*H-*azepinyl, hexahydro-1*H-*1,4-diazepinyl, hexahydro-1,4-oxazepinyl, 1,2,3,4- tetrahydroisoquinolin-2-yl, 2,5-diazabicyclo[2.2.1]heptyl, pyrrolinyl, pyrrolyl, imidazolidinyl, pyrazolidinyl, 2-imidazolinyl, 2-pyrazolinyl, imidazolyl, pyrazolyl, triazolyl, pyridinyl, pyridazinyl, pyrimidinyl, pyrazinyl and triazinyl, each radical optionally substituted with 1, 2, 3 or 4 substituents, each substituent independently selected from C₁₋₆alkyl, haloC₁₋₆alkyl, C₁₋₆alkylcarbonyl, halo, arylC₁₋₆alkyl, hydroxy, C₁₋₆alkyloxy, amino, mono- or diC₁₋₆alkylamino, C₁₋₆alkylthio, C₁₋₆alkylthioC₁₋₆alkyl, aryl, pyridyl, pyrimidinyl, piperidinyl or pyrrolidinyl optionally substituted with arylC₁₋₆alkyl;
- R^{4a} and R^{5a}: together with the nitrogen atom to which they are attached form a radical selected from the group consisting of pyrrolidino, piperidino, piperazino, morpholino, 4-thiomorpholino, 2,3-dihydroisoindol-1-yl, thiazolidin-3-yl, 1,2,3,6-tetrahydropyridyl, hexahydro-1*H-*azepinyl, hexahydro-1*H-*1,4-diazepinyl, hexahydro-1,4-oxazepinyl, 1,2,3,4- tetrahydroisoquinolin-2-yl, pyrrolinyl, pyrrolyl, imidazolidinyl, pyrazolidinyl, 2-imidazolinyl, 2-pyrazolinyl, imidazolyl, pyrazolyl, triazolyl, pyridinyl, pyridazinyl, pyrimidinyl, pyrazinyl and triazinyl, each radical optionally substituted with 1, 2, 3 or 4 substituents, each substituent independently selected from C₁₋₆alkyl, haloC₁₋₆alkyl, halo, arylC₁₋₆alkyl, hydroxy, C₁₋₆alkyloxy, amino, mono- or diC₁₋₆alkylamino, C₁₋₆alkylthio, C₁₋₆alkylthioC₁₋₆alkyl, aryl, pyridyl or pyrimidinyl;
- R⁷: is hydrogen, halo, C₁₋₆alkyl, aryl or Het;
- R⁸: is hydrogen or C₁₋₆alkyl;
- R⁹: is oxo; or
- R⁸ and R⁹: together form the radical -CH=CH-N=;
- R¹¹: is hydrogen or C₁₋₆alkyl;
- aryl: is a homocycle selected from phenyl, naphthyl, acenaphthyl or tetrahydronaphthyl, each being optionally substituted with 1, 2 or 3 substituents, each substituent being independently selected from hydroxy, halo, cyano, nitro, amino, mono- or diC₁₋₆alkylamino, C₁₋₆alkyl, haloC₁₋₆alkyl, C₁₋₆alkyloxy, haloC₁₋₆alkyloxy, carboxyl, C₁₋₆alkyloxycarbonyl, aminocarbonyl, morpholinyl or mono- or diC₁₋₆alkylaminocarbonyl;
- Het: is a monocyclic heterocycle selected from *N-*phenoxypiperidinyl, piperidinyl, pyrrolyl, pyrazolyl, imidazolyl, furanyl, thienyl, oxazolyl, isoxazolyl, thiazolyl, isothiazolyl, pyridinyl, pyrimidinyl, pyrazinyl or pyridazinyl; or a bicyclic heterocycle selected from quinolinyl, quinoxalinyl, indolyl, benzimidazolyl, benzoxazolyl, benzisoxazolyl, benzothiazolyl, benzisothiazolyl, benzofuranyl, benzothienyl, 2,3-dihydrobenzo[1,4]dioxinyl or benzo[1,3]dioxolyl; each monocyclic and bicyclic heterocycle being optionally substituted with 1, 2 or 3 substituents, each substituent independently selected from halo, hydroxy, C₁₋₆alkyl or C₁₋₆alkyloxy.

A fourth interesting embodiment relates to a compound of formula (Ia) or (Ib) or any subgroup thereof as mentioned hereinbefore as interesting embodiment wherein R¹ is hydrogen, cyano, halo, alkyl, haloalkyl, hydroxy, alkyloxy, alkylthio, alkyloxyalkyl, alkylthioalkyl, arylalkyl, di(aryl)alkyl, aryl, or Het; in particular R¹ is halo, aryl or Het; more in particular R¹ is halo. Most preferably, R¹ is bromo. Or R¹ represents formyl, carboxyl, C₂₋₆alkenyl, C₂-₆alkynyl, -C=N-OR¹¹, amino, mono or di(alkyl)amino, aminoalkyl, mono or di(alkyl)aminoalkyl, alkylcarbonylaminoalkyl, aminocarbonyl, mono or di(alkyl)aminocarbonyl, arylcarbonyl, R^{5a}R^{4a}Nalkyl, R^{5a}R^{4a}N-, R^{5a}R^{4a}N-C(=O)-.

A fifth interesting embodiment relates to a compound of formula (Ia) or (Ib) or any subgroup thereof as mentioned hereinbefore as interesting embodiment wherein p is equal to 1.

A sixth interesting embodiment relates to a compound of formula (Ia) or (Ib) or any subgroup thereof as mentioned hereinbefore as interesting embodiment wherein R² is hydrogen, alkyloxy or alkylthio, in particular hydrogen, C₁₋₆alkyloxy or C₁₋₆alkylthio. More in particular, R² is C₁₋₆alkyloxy, preferably methyloxy.

A seventh interesting embodiment relates to a compound of formula (Ia) or (Ib) or any subgroup thereof as mentioned hereinbefore as interesting embodiment wherein R³ is C₁₋₆alkyl, C₃₋₆cycloalkyl, arylC₁₋₆alkyl, aryl, Het, Het-C₁₋₆alkyl; in particular aryl or arylC₁₋₆alkyl; more in particular aryl, such as optionally substituted phenyl or optionally substituted naphthyl; even more in particular naphthyl. Or R³ is aryl-O-C₁₋₆alkyl, arylC₁₋₆alkyl-O-C₁₋₆alkyl, aryl-aryl, Het-O-C₁₋₆alkyl, HetC₁₋₆alkyl-O-C₁₋₆alkyl, or 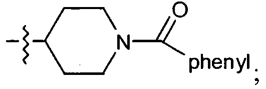 or R³ is aryl-O-C₁₋₆alkyl, arylC₁₋₆alkyl-O-C₁₋₆alkyl, Het-O-C₁₋₆alkyl, HetC₁₋₆alkyl-O-C₁₋₆alkyl, or

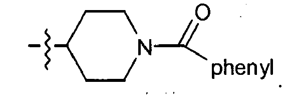

An eighth interesting embodiment relates to a compound of formula (Ia) or (Ib) or any subgroup thereof as mentioned hereinbefore as interesting embodiment wherein q is equal to 2, 3 or 4.

A ninth interesting embodiment relates to a compound of formula (Ia) or (Ib) or any subgroup thereof as mentioned hereinbefore as interesting embodiment wherein R⁴ and R⁵ each independently represent hydrogen or C₁₋₆alkyl, in particular C₁₋₆alkyl, more in particular methyl or ethyl. Preferably R⁴ and R⁵ are methyl.

A tenth interesting embodiment relates to a compound of formula (Ia) or (Ib) or any subgroup thereof as mentioned hereinbefore as interesting embodiment wherein R⁴ and R⁵ together with the nitrogen atom to which they are attached form a radical selected from the group consisting of piperidino, piperazino, morpholino, imidazolyl, triazolyl, each of said rings optionally substituted with C₁₋₆alkyl; more in particular piperidino or piperazino, each of said rings optionally substituted with C₁₋₆alkyl; even more in particular piperidino or piperazino optionally substituted with C₁₋₆alkyl.

An eleventh interesting embodiment relates to a compound of formula (Ia) or (Ib) or any subgroup thereof as mentioned hereinbefore as interesting embodiment wherein R⁷ is hydrogen.

A twelfth interesting embodiment relates to a compound of formula (Ia) or (Ib) or any subgroup thereof as mentioned hereinbefore as interesting embodiment wherein the compound is a compound of formula (Ia).

A thirteenth interesting embodiment relates to a compound of formula (Ia) or (Ib) or any subgroup thereof as mentioned hereinbefore as interesting embodiment wherein the compound is a compound of formula (Ib) and wherein R⁸ is hydrogen and R⁹ is oxo.

A fourteenth interesting embodiment relates to a compound of formula (Ia) or (Ib) or any subgroup thereof as mentioned hereinbefore as interesting embodiment wherein the compound is a compound of formula (Ib), in particular wherein R⁸ is alkyl, more preferable C₁₋₆alkyl, e.g. methyl.

A fifteenth interesting embodiment is a compound of formula (Ia) or (Ib) or any subgroup thereof as mentioned hereinbefore as interesting embodiment wherein aryl is naphthyl or phenyl, more preferably phenyl, each optionally substituted with one or two substituents selected from halo, for example chloro; cyano; alkyl for example methyl; or alkyloxy, for example methyloxy.

A sixteenth interesting embodiment relates to a compound of formula (Ia) or (Ib) or any subgroup thereof as mentioned hereinbefore as interesting embodiment wherein R¹ is placed in position 6 of the quinoline ring.
In the framework of this application, the quinoline ring of the compounds of formula (Ia) or (Ib) is numbered as follows :

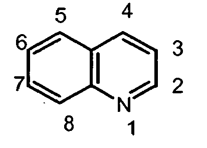

A seventeenth interesting embodiment is the use of a compound of formula (Ia) or (Ib) or any subgroup thereof as mentioned hereinbefore as interesting embodiment for the manufacture of a medicament for the treatment of a bacterial infection with a gram-positive and/or a gram-negative bacterium, preferably a bacterial infection with a gram-positive bacterium.

An eighteenth interesting embodiment is the use of a compound of formula (Ia) or (Ib) or any subgroup thereof as mentioned hereinbefore as interesting embodiment for the manufacture of a medicament for the treatment of a bacterial infection wherein the compound of formula (Ia) or (Ib) has a IC₉₀ < 15 µl/ml against at least one bacterium, in particular a gram-positive bacterium; preferably a IC₉₀ < 10 µl/ml; more preferably a IC₉₀ < 5 µl/ml; the IC₉₀ value being determined as described hereinafter.

A nineteenth interesting embodiment relates to a compound of formula (Ia) or any subgroup thereof as mentioned hereinbefore as interesting embodiment wherein one or more, preferably all, of the following definitions apply :
R¹ is halo, preferably bromo;
R² is C₁₋₆alkyloxy, preferably methyloxy;
R³ is aryl, in particular naphthyl;
R⁴ and R⁵ are C₁₋₆alkyl; in particular methyl;
R⁷ is hydrogen;
q is 2, 3 or 4;
p is 1.

Preferably, in the compounds of formula (Ia) and (Ib) or any subgroup thereof as mentioned hereinbefore as interesting embodiment, the term "alkyl" represents C₁₋₆alkyl, more preferably C₁₋₄alkyl, and the term haloalkyl represents polyhaloC₁₋₆alkyl.

### PHARMACOLOGY

The compounds according to the invention have surprisingly been shown to be suitable for the treatment of a bacterial infection including a mycobacterial infection, particularly those diseases caused by pathogenic mycobacteria such as *Mycobacterium tuberculosis* (including the latent and drug resistant form thereof), *M. bovis, M. avium, M. leprae* and *M*. *marinum.* The present invention thus also relates to compounds of formula (Ia) or (Ib) as defined hereinabove, the pharmaceutically acceptable salts thereof or the *N-*oxide forms thereof or the solvates thereof, for use as a medicine, in particular for use as a medicine for the treatment of a bacterial infection including a mycobacterial infection.

Further, the present invention also relates to the use of a compound of formula (Ia) or (Ib), the pharmaceutically acceptable salts thereof or the *N-*oxide forms thereof or the solvates thereof, as well as any of the pharmaceutical compositions thereof as described hereinafter for the manufacture of a medicament for the treatment of a bacterial infection including a mycobacterial infection.

Accordingly, in another aspect, the invention provides a method of treating a patient suffering from, or at risk of, a bacterial infection, including a mycobacterial infection, which comprises administering to the patient a therapeutically effective amount of a compound or pharmaceutical composition according to the invention.

In addition to their activity against mycobacteria, the compounds according to the invention are also active against other bacteria. In general, bacterial pathogens may be classified as either gram-positive or gram-negative pathogens. Antibiotic compounds with activity against both gram-positive and gram-negative pathogens are generally regarded as having a broad spectrum of activity. The compounds of the present invention are regarded as active against gram-positive and/or gram-negative bacterial pathogens, in particular against gram-positive bacterial pathogens. In particular, the present compounds are active against at least one gram-positive bacterium, preferably against several gram-positive bacteria, more preferably against one or more gram-positive bacteria and/or one or more gram-negative bacteria.

The present compounds have bactericidal or bacteriostatic activity.

Examples of gram-positive and gram-negative aerobic and anaerobic bacteria, include Staphylococci, for example *S. aureus;* Enterococci, for example *E. faecalis;* Streptococci, for example *S. pneumoniae, S. mutans, S. pyogens;* Bacilli, for example *Bacillus subtilis;* Listeria, for example *Listeria monocytogenes;* Haemophilus, for example *H. influenza;* Moraxella, for example *M. catarrhalis;* Pseudomonas, for example *Pseudomonas aeruginosa;* and Escherichia, for example *E. coli.* Gram-positive pathogens, for example Staphylococci, Enterococci and Streptococci are particularly important because of the development of resistant strains which are both difficult to treat and difficult to eradicate from for example a hospital environment once established. Examples of such strains are methicillin resistant *Staphylococcus aureus* (MRSA), methicillin resistant coagulase negative staphylococci (MRCNS), penicillin resistant *Streptococcus pneumoniae* and multiple resistant *Enterococcus faecium.*

The compounds of the present invention also show activity against resistant bacterial strains.

The compounds of the present invention are especially active against *Streptococcus pneumoniae* and *Staphylococcus aureus,* including resistant *Staphylococcus aureus* such as for example methicillin resistant *Staphylococcus aureus* (MRSA).

Therefore, the present invention also relates to the use of a compound of formula (Ia) or (Ib), the pharmaceutically acceptable salts thereof or the *N-*oxide forms thereof or the solvates thereof, as well as any of the pharmaceutical compositions thereof as described hereinafter for the manufacture of a medicament for the treatment of a bacterial infection including an infection caused by Staphylococci and/or Streptococci.

Accordingly, in another aspect, the invention provides a method of treating a patient suffering from, or at risk of, a bacterial infection, including an infection caused by Staphylococci and/or Streptococci, which comprises administering to the patient a therapeutically effective amount of a compound or pharmaceutical composition according to the invention.

Without being bound to any theory, it is taught that the activity of the present compounds lies in inhibition of the F1F0 ATP synthase, in particular the inhibition of the F0 complex of the F1F0 ATP synthase, more in particular the inhibition of subunit c of the F0 complex of the F1F0 ATP synthase, leading to killing of the bacteria by depletion of the cellular ATP levels of the bacteria. Therefore, in particular, the compounds of the present invention are active on those bacteria of which the viability depends on proper functioning of F1F0 ATP synthase.

Bacterial infections which may be treated by the present compounds include, for example, central nervous system infections, external ear infections, infections of the middle ear, such as acute otitis media, infections of the cranial sinuses, eye infections, infections of the oral cavity, such as infections of the teeth, gums and mucosa, upper respiratory tract infections, lower respiratory tract infections, genitourinary infections, gastrointestinal infections, gynaecological infections, septicemia, bone and joint infections, skin and skin structure infections, bacterial endocarditis, bums, antibacterial prophylaxis of surgery, and antibacterial prophylaxis in immunosuppressed patients, such as patients receiving cancer chemotherapy, or organ transplant patients.

Whenever used hereinbefore or hereinafter, that the compounds can treat a bacterial infection it is meant that the compounds can treat an infection with one or more bacterial strains.

The invention also relates to a composition comprising a pharmaceutically acceptable carrier and, as active ingredient, a therapeutically effective amount of a compound according to the invention. The compounds according to the invention may be formulated into various pharmaceutical forms for administration purposes. As appropriate compositions there may be cited all compositions usually employed for systemically administering drugs. To prepare the pharmaceutical compositions of this invention, an effective amount of the particular compound, optionally in addition salt form, as the active ingredient is combined in intimate admixture with a pharmaceutically acceptable carrier, which carrier may take a wide variety of forms depending on the form of preparation desired for administration. These pharmaceutical compositions are desirable in unitary dosage form suitable, in particular, for administration orally or by parenteral injection. For example, in preparing the compositions in oral dosage form, any of the usual pharmaceutical media may be employed such as, for example, water, glycols, oils, alcohols and the like in the case of oral liquid preparations such as suspensions, syrups, elixirs, emulsions and solutions; or solid carriers such as starches, sugars, kaolin, diluents, lubricants, binders, disintegrating agents and the like in the case of powders, pills, capsules and tablets. Because of their ease in administration, tablets and capsules represent the most advantageous oral dosage unit forms in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. Also included are solid form preparations which are intended to be converted, shortly before use, to liquid form preparations.

Depending on the mode of administration, the pharmaceutical composition will preferably comprise from 0.05 to 99 % by weight, more preferably from 0.1 to 70 % by weight, even more preferably from 0.1 to 50 % by weight of the active ingredient(s), and, from 1 to 99.95 % by weight, more preferably from 30 to 99.9 % by weight, even more preferably from 50 to 99.9 % by weight of a pharmaceutically acceptable carrier, all percentages being based on the total weight of the composition.

The pharmaceutical composition may additionally contain various other ingredients known in the art, for example, a lubricant, stabilising agent, buffering agent, emulsifying agent, viscosity-regulating agent, surfactant, preservative, flavouring or colorant.

It is especially advantageous to formulate the aforementioned pharmaceutical compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association with the required pharmaceutical carrier. Examples of such unit dosage forms are tablets (including scored or coated tablets), capsules, pills, powder packets, wafers, suppositories, injectable solutions or suspensions and the like, and segregated multiples thereof.
The daily dosage of the compound according to the invention will, of course, vary with the compound employed, the mode of administration, the treatment desired and the mycobacterial disease indicated. However, in general, satisfactory results will be obtained when the compound according to the invention is administered at a daily dosage not exceeding 1 gram, e.g. in the range from 10 to 50 mg/kg body weight.

Given the fact that the compounds of formula (Ia) or Formula (Ib) are active against bacterial infections, the present compounds may be combined with other antibacterial agents in order to effectively combat bacterial infections.

Therefore, the present invention also relates to a combination of (a) a compound according to the invention, and (b) one or more other antibacterial agents.

The present invention also relates to a combination of (a) a compound according to the invention, and (b) one or more other antibacterial agents, for use as a medicine.

The present invention also relates to the use of a combination or pharmaceutical composition as defined directly above for the treatment of a bacterial infection.

A pharmaceutical composition comprising a pharmaceutically acceptable carrier and, as active ingredient, a therapeutically effective amount of (a) a compound according to the invention, and (b) one or more other antibacterial agents, is also comprised by the present invention.

The weight ratio of (a) the compound according to the invention and (b) the other antibacterial agent(s) when given as a combination may be determined by the person skilled in the art. Said ratio and the exact dosage and frequency of administration depends on the particular compound according to the invention and the other antibacterial agent(s) used, the particular condition being treated, the severity of the condition being treated, the age, weight, gender, diet, time of administration and general physical condition of the particular patient, the mode of administration as well as other medication the individual may be taking, as is well known to those skilled in the art. Furthermore, it is evident that the effective daily amount may be lowered or increased depending on the response of the treated subject and/or depending on the evaluation of the physician prescribing the compounds of the instant invention. A particular weight ratio for the present compound of formula (Ia) or (Ib) and another antibacterial agent may range from 1/10 to 10/1, more in particular from 1/5 to 5/1, even more in particular from 1/3 to 3/1.

The compounds according to the invention and the one or more other antibacterial agents may be combined in a single preparation or they may be formulated in separate preparations so that they can be administered simultaneously, separately or sequentially. Thus, the present invention also relates to a product containing (a) a compound according to the invention, and (b) one or more other antibacterial agents, as a combined preparation for simultaneous, separate or sequential use in the treatment of a bacterial infection.

The other antibacterial agents which may be combined with the compounds of formula (Ia) or (Ib) are for example antibacterial agents known in the art. The other antibacterial agents comprise antibiotics of the β-lactam group such as natural penicillins, semisynthetic penicillins, natural cephalosporins, semisynthetic cephalosporins, cephamycins, 1-oxacephems, clavulanic acids, penems, carbapenems, nocardicins, monobactams; tetracyclines, anhydrotetracyclines, anthracyclines; aminoglycosides; nucleosides such as *N-*nucleosides, C-nucleosides, carbocyclic nucleosides, blasticidin S; macrolides such as 12-membered ring macrolides, 14-membered ring macrolides, 16-membered ring macrolides; ansamycins; peptides such as bleomycins, gramicidins, polymyxins, bacitracins, large ring peptide antibiotics containing lactone linkages, actinomycins, amphomycin, capreomycin, distamycin, enduracidins, mikamycin, neocarzinostatin, stendomycin, viomycin, virginiamycin; cycloheximide; cycloserine; variotin; sarkomycin A; novobiocin; griseofulvin; chloramphenicol; mitomycins; fumagillin; monensins; pyrrolnitrin; fosfomycin; fusidic acid; D-(p-hydroxyphenyl)glycine; D-phenylglycine; enediynes.

Specific antibiotics which may be combined with the present compounds of formula (Ia) or (Ib) are for example benzylpenicillin (potassium, procaine, benzathine), phenoxymethylpenicillin (potassium), phenethicillin potassium, propicillin, carbenicillin (disodium, phenyl sodium, indanyl sodium), sulbenicillin, ticarcillin disodium, methicillin sodium, oxacillin sodium, cloxacillin sodium, dicloxacillin, flucloxacillin, ampicillin, mezlocillin, piperacillin sodium, amoxicillin, ciclacillin, hectacillin, sulbactam sodium, talampicillin hydrochloride, bacampicillin hydrochloride, pivmecillinam, cephalexin, cefaclor, cephaloglycin, cefadroxil, cephradine, cefroxadine, cephapirin sodium, cephalothin sodium, cephacetrile sodium, cefsulodin sodium, cephaloridine, cefatrizine, cefoperazone sodium, cefamandole, vefotiam hydrochloride, cefazolin sodium, ceftizoxime sodium, cefotaxime sodium, cefmenoxime hydrochloride, cefuroxime, ceftriaxone sodium, ceftazidime, cefoxitin, cefmetazole, cefotetan, latamoxef, clavulanic acid, imipenem, aztreonam, tetracycline, chlortetracycline hydrochloride, demethylchlortetracycline, oxytetracycline, methacycline, doxycycline, rolitetracycline, minocycline, daunorubicin hydrochloride, doxorubicin, aclarubicin, kanamycin sulfate, bekanamycin, tobramycin, gentamycin sulfate, dibekacin, amikacin, micronomicin, ribostamycin, neomycin sulfate, paromomycin sulfate, streptomycin sulfate, dihydrostreptomycin, destomycin A, hygromycin B, apramycin, sisomicin, netilmicin sulfate, spectinomycin hydrochloride, astromicin sulfate, validamycin, kasugamycin, polyoxin, blasticidin S, erythromycin, erythromycin estolate, oleandomycin phosphate, tracetyloleandomycin, kitasamycin, josamycin, spiramycin, tylosin, ivermectin, midecamycin, bleomycin sulfate, peplomycin sulfate, gramicidin S, polymyxin B, bacitracin, colistin sulfate, colistinmethanesulfonate sodium, enramycin, mikamycin, virginiamycin, capreomycin sulfate, viomycin, enviomycin, vancomycin, actinomycin D, neocarzinostatin, bestatin, pepstatin, monensin, lasalocid, salinomycin, amphotericin B, nystatin, natamycin, trichomycin, mithramycin, lincomycin, clindamycin, clindamycin palmitate hydrochloride, flavophospholipol, cycloserine, pecilocin, griseofulvin, chloramphenicol, chloramphenicol palmitate, mitomycin C, pyrrolnitrin, fosfomycin, fusidic acid, bicozamycin, tiamulin, siccanin.

Other Mycobacterial agents which may be combined with the compounds of formula (Ia) or (Ib) are for example rifampicin (=rifampin); isoniazid; pyrazinamide; amikacin; ethionamide; ethambutol; streptomycin; para-aminosalicylic acid; cycloserine; capreomycin; kanamycin; thioacetazone; PA-824; quinolones/fluoroquinolones such as for example moxifloxacin, gatifloxacin, ofloxacin, ciprofloxacin, sparfloxacin; macrolides such as for example clarithromycin, clofazimine, amoxycillin with clavulanic acid; rifamycins; rifabutin; rifapentine; the compounds disclosed in WO2004/011436.

### GENERAL PREPARATION

The compounds according to the invention can generally be prepared by a succession of steps, each of which is known to the skilled person.

In particular, the compounds of formula (Ia) or (Ib) can be prepared by reacting an intermediate of formula (IIa) or (IIb) with an intermediate of formula (III) according to the following reaction scheme (1) : 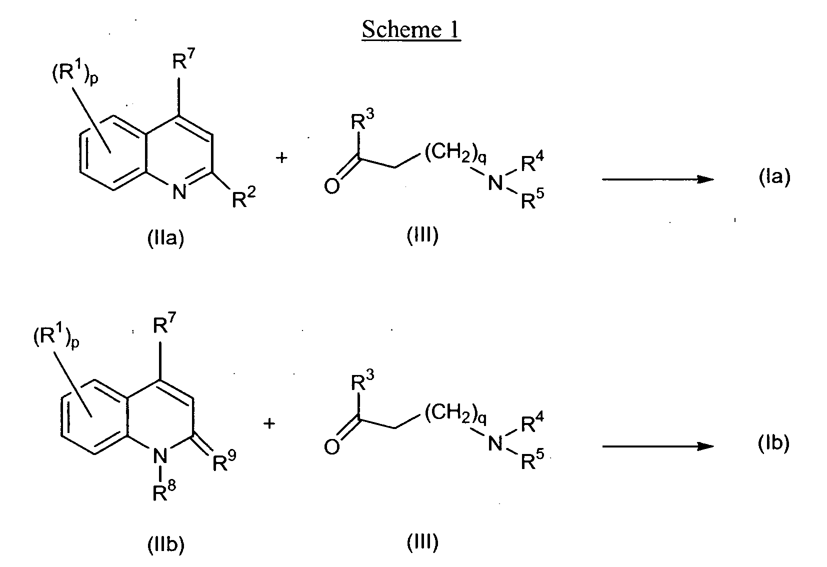 using nBuLi in a mixture of a suitable base, such as for example 2,2,6,6-tetramethylpiperidine or diisopropyl amine, and a suitable solvent, such as for example tetrahydrofuran, wherein all variables are defined as in formula (Ia) or (Ib). Stirring may enhance the rate of the reaction. The reaction may conveniently be carried out at a temperature ranging between -20 and -70 °C.

Compounds of formula (Ia) or (Ib) can also be prepared by reacting an intermediate of formula (IV-a) or (IV-b) wherein W₂ represents a suitable leaving group, such as for example halo, e.g. chloro or bromo, with a suitable primary or secondary amine HNR⁴R⁵.

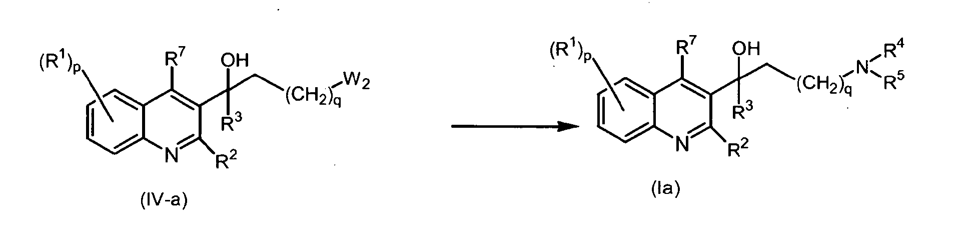

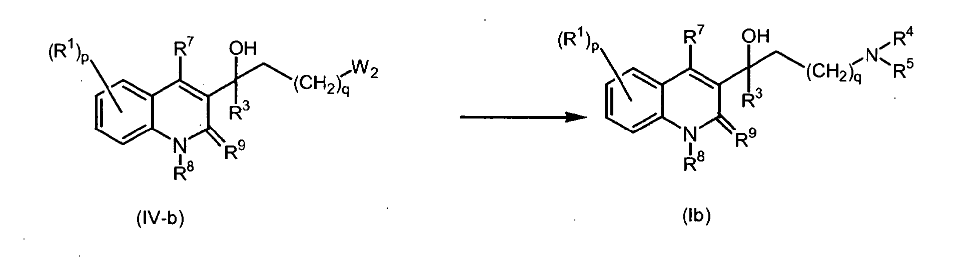

It is considered within the knowledge of the skilled man to explore the appropriate temperatures, dilutions, and reaction times in order to optimize the above reactions in order to obtain a desired compound.

The compounds of formula (Ia) or (Ib) may further be prepared by converting compounds of formula (Ia) or (Ib) into each other according to art-known group transformation reactions.

The compounds of formula (Ia) or (Ib) may be converted to the corresponding *N-*oxide forms following art-known procedures for converting a trivalent nitrogen into its *N-*oxide form. Said *N-*oxidation reaction may generally be carried out by reacting the starting material of formula (Ia) or (Ib) with an appropriate organic or inorganic peroxide. Appropriate inorganic peroxides comprise, for example, hydrogen peroxide, alkali metal or earth alkaline metal peroxides, e.g. sodium peroxide, potassium peroxide; appropriate organic peroxides may comprise peroxy acids such as, for example, benzenecarboperoxoic acid or halo substituted benzenecarboperoxoic acid, e.g. 3-chlorobenzenecarboperoxoic acid, peroxoalkanoic acids, e.g. peroxoacetic acid, alkylhydroperoxides, e.g. tert.butyl hydro-peroxide. Suitable solvents are, for example, water, lower alcohols, e.g. ethanol and the like, hydrocarbons, e.g. toluene, ketones, e.g. 2-butanone, halogenated hydrocarbons, e.g. dichloromethane, and mixtures of such solvents.

Compounds of formula (Ia) or (Ib) wherein R¹ represents halo, e.g. bromo, can be converted into a compound of formula (Ia) or (Ib) wherein R¹ represents aryl or Het, by reaction with aryl-B(OH)₂ respectively Het-B(OH)₂ in the presence of a suitable catalyst, such as for example Pd(OAc)₂ or Pd(PPh₃)₄, in the presence of a suitable base, such as for example K₃P0₄ or Na₂CO₃, and a suitable solvent, such as for example toluene, an alcohol, e.g. methanol, or 1,2-dimethoxyethane (DME).

Similarly, compounds of formula (Ia) or (Ib) in which R¹ is halo, for example bromo, may be converted into compounds of formula (Ia) or (Ib) in which R¹ is alkyl, for example methyl, by treatment with an appropriate alkylating agent such as CH₃B(OH)₂ or (CH₃)₄Sn in the presence of a suitable catalyst, such as for example Pd(PPh₃)₄, in a suitable solvent such as for example toluene or 1,2-dimethoxyethane (DME).

Compounds of formula (Ia) or (Ib) wherein R¹ is halo, in particular bromo, can be converted into a compound of formula (Ia) or (Ib) wherein R¹ is hydrogen, by reaction with HCOONH₄ in the presence of a suitable catalyst such as for example palladium on charcoal, and in the presence of a suitable solvent, such as for example an alcohol, e.g. methanol. The same reaction conditions can be used to convert a compound of formula (Ia) or (Ib) wherein R⁴ is benzyl into a compound of formula (Ia) or (Ib) wherein R⁴ is hydrogen.

Compounds of formula (Ia) or (Ib) wherein R¹ is halo, in particular bromo, can also be converted into a compound wherein R¹ is formyl by reaction with *N,N*-dimethylformamide in the presence of nBuLi and a suitable solvent, such as for example tetrahydrofuran. These compounds can then further be converted into a compound of formula (Ia) or (Ib) wherein R¹ is -CH₂-OH by reaction with a suitable reducing agent, such as for example NaBH₄ and in the presence of a suitable solvent, such as for example an alcohol, e.g. methanol, and tetrahydrofuran.

Compounds of formula (Ia) or (Ib) wherein R¹ represents C₂₋₆alkenyl, can be prepared by reacting a compound of formula (Ia) or (Ib) wherein R¹ is halo, e.g. bromo and the like, with tributyl(C₂₋₆alkenyl)tin, such as for example tributyl(vinyl)tin, in the presence of a suitable catalyst, such as for example Pd(PPh₃)₄, in the presence of a suitable solvent, such as for example *N,N*-dimethylformamide. This reaction is preferably performed at elevated temperature.

Compounds of formula (Ia) or (Ib) wherein R¹ represents R^{5a}R^{4a}N-, can be prepared from a compound of formula (Ia) or (Ib) wherein R¹ is halo, e.g. bromo and the like, by reaction with R^{5a}R^{4a}NH in the presence of a suitable catalyst, such as for example tris(dibenzylideneacetone)palladium, a suitable ligand, such as for example 2-(di-t-butylphosphino)biphenyl, a suitable base, such as for example sodium t-butoxide, and a suitable solvent, such as for example toluene.

Compounds of formula (Ia) or (Ib) wherein R¹ represents -C=N-OR¹¹, can be prepared from a compound of formula (Ia) or (Ib) wherein R¹ is formyl, by reaction with hydroxylamine hydrochloride or C₁₋₆alkoxylamine hydrochloride in the presence of a suitable solvent, such as for example pyridine.

Compounds of formula (Ia) or (Ib) wherein R¹ represents -CH₂-NH₂, can be prepared from a compound of formula (Ia) or (Ib) wherein R¹ is formyl, by reduction in the presence of H₂, a suitable catalyst, such as for example palladium on charcoal, and a suitable solvent, such as for example NH₃/alcohol, e.g. NH₃/methanol. Compounds of formula (Ia) or (Ib) wherein R¹ represents -CH₂-NH₂ can be converted into a compound of formula (Ia) or (Ib) wherein R¹ represents -CH₂-N(C₁₋₆alkyl)₂ by reaction with a suitable aldehyde or ketone reagent, such as for example paraformaldehyde or formaldehyde, in the presence of sodium cyanoborohydride, acetic acid and a suitable solvent, such as for example acetonitrile.

Compounds of formula (Ia) or (Ib) wherein R¹ represents R^{5a}R^{4a}N-CH₂-, can be prepared by reacting a compound of formula (Ia) or (Ib) wherein R¹ is formyl, with a suitable reagent of formula R^{5a}R^{4a}N-H in the presence of a suitable reducing agent, such as for example BH₃CN, a suitable solvent, such as for example acetonitrile and tetrahydrofuran, and a suitable acid, such as for example acetic acid.

Compounds of formula (Ia) or (Ib) wherein R¹ represents amino, can be prepared by reacting a compound of formula (Ia) or (Ib) wherein R¹ is carboxyl, with a suitable azide, such as for example diphenylphosphorylazide (DPPA), and a suitable base, such as for example triethylamine, in a suitable solvent, such as for example toluene. The obtained product undergoes a Curtius reaction, and by adding trimethylsilylethanol a carbamate intermediate is formed. In a next step, this intermediate is reacted with tetrabutylammonium bromide (TBAB) in a suitable solvent, such as for example tetrahydrofuran to obtain the amino derivative.

Compounds of formula (Ia) or (Ib) wherein R¹ represents aminocarbonyl, mono or di(alkyl)aminocarbonyl or R^{5a}R^{4a}N-C(=O)-, can be prepared by reacting a compound of formula (Ia) or (Ib) wherein R¹ is carboxyl, with a suitable amine, a suitable coupling reagent such as for example hydroxybenzotriazole, a suitable activating reagent such as for example 1,1'-carbonyldiimidazole or *N,N'*-dicyclohexylcarbodiimide or 1-(3-dimethylaminopropyl)-3-ethylcarbodiimide, a suitable base, such as for example triethylamine, and a suitable solvent, such as for example tetrahydrofuran and methylenechloride.

Compounds of formula (Ia) or (Ib) wherein R¹ represents arylcarbonyl, can be prepared by reacting in a first step (a) a compound of formula (Ia) or (Ib) wherein R¹ is halo, e.g. bromo and the like, with a suitable arylaldehyde in the presence of nBuLi and a suitable solvent, such as for example tetrahydrofuran. This reaction is preferably performed at low temperature such as for example -70°C. In a next step (b), the product obtained in step (a) is oxidized with a suitable oxidans, such as for example manganese oxide, in the presence of a suitable solvent, such as for example methylene chloride.

Compounds of formula (Ia) or (Ib) wherein R⁴ and R⁵ represent a ring moiety substituted with alkylcarbonyl, can be prepared from the corresponding compound wherein the ring moiety is unsubstituted by reaction with an appropriate acyl chloride, e.g. acetyl chloride, in the presence of a suitable base, such as for example triethylamine, and a suitable solvent, such as for example methylene chloride.

Compounds of formula (Ia) or (Ib) wherein R⁴ and R⁵ represent an unsubstituted ring moiety, can be prepared from the corresponding compound wherein the ring moiety is substituted with arylalkyl, by reaction with ammonium formate in the presence of a suitable catalyst, such as for example palladium on charcoal, and a suitable solvent, such as for example an alcohol, e.g. methanol.

A compound of formula (Ia) wherein R² represents methoxy, can be converted into the corresponding compound of fomula (Ib) wherein R⁸ is hydrogen and R⁹ is oxo, by hydrolysis in the presence of a suitable acid, such as for example hydrochloric acid, and a suitable solvent, such as for example dioxane.

Compounds of formula (Ia) or (Ib) wherein R⁴ and R⁵ are taken together with the nitrogen to which they are attached to form 1,1-dioxide-thiomorpholinyl, can be prepared from the corresponding thiomorpholine derivative by reaction with an appropriate organic or inorganic peroxide. Appropriate inorganic peroxides comprise, for example, hydrogen peroxide, alkali metal or earth alkaline metal peroxides, e.g. sodium peroxide, potassium peroxide; appropriate organic peroxides may comprise peroxy acids such as, for example, benzenecarboperoxoic acid or halo substituted benzenecarboperoxoic acid, e.g. 3-chlorobenzenecarboperoxoic acid, peroxoalkanoic acids, e.g. peroxoacetic acid, alkylhydroperoxides, e.g. tert.butyl hydro-peroxide. Suitable solvents are, for example, water, lower alcohols, e.g. ethanol and the like, hydrocarbons, e.g. toluene, ketones, e.g. 2-butanone, halogenated hydrocarbons, e.g. dichloromethane, and mixtures of such solvents.

Compounds of formula (Ia) or (Ib) can also be converted into a quaternary amine by reaction with a suitable quaternizing agent, such as, for example, an optionally substituted C₁₋₆alkylhalide, arylC₁₋₆alkylhalide, C₁₋₆alkylcarbonylhalide, arylcarbonylhalide, Het¹C₁₋₆alkylhalide or Het¹carbonylhalide, e.g. methyliodide or benzyliodide, in the presence of a suitable solvent, such as for example acetone wherein Het¹ represents furanyl or thienyl; or a bicyclic heterocycle selected from benzofuranyl or benzothienyl; each monocyclic and bicyclic heterocycle may optionally be substituted with 1, 2 or 3 substituents, each substituent independently selected from the group of halo, C₁₋₆alkyl and aryl. Said quaternary amines are represented by the below formula wherein R¹⁰ represents C₁₋₆alkyl, C₁₋₆alkylcarbonyl, arylC₁₋₆alkyl, arylcarbonyl, Het¹C₁₋₆alkyl or Het¹carbonyl and wherein A⁻ represents a pharmaceutically acceptable counter ion, such as for example iodide.

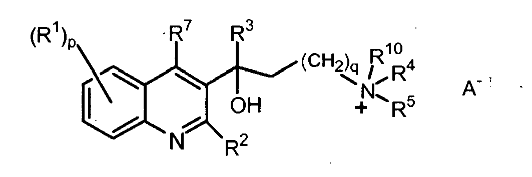

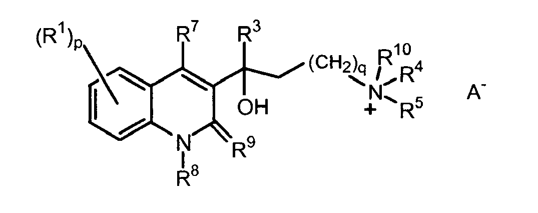

It is evident that in the foregoing and in the following reactions, the reaction products may be isolated from the reaction medium and, if necessary, further purified according to methodologies generally known in the art, such as extraction, crystallization and chromatography. It is further evident that reaction products that exist in more than one enantiomeric form, may be isolated from their mixture by known techniques, in particular preparative chromatography, such as preparative HPLC, chiral chromatography. Individual diastereoisomers or individual enantiomers can also be obtained by Supercritical Fluid Chromatography (SCF).

Intermediates of formula (IIa) or or intermediates of formula (IIb) wherein R⁸ is hydrogen, said intermediates being represented by formula (IIa-1), (IIa-2), (IIa-3), (IIa-4), or (IIb-1), may be prepared according to the following reaction scheme (2): 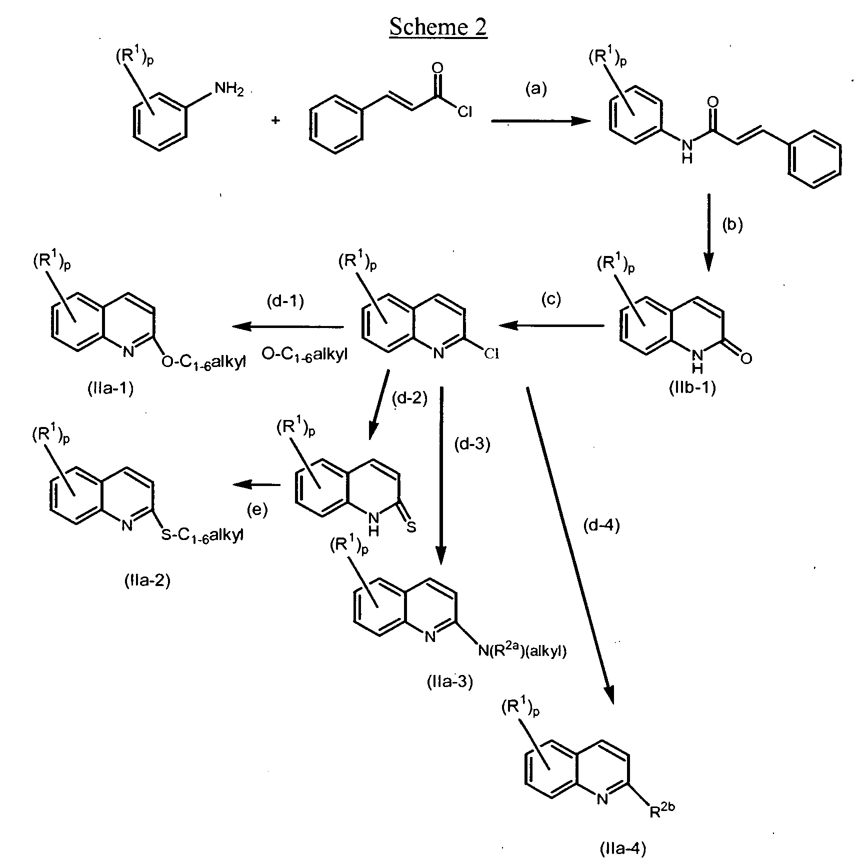 wherein all variables are defined as in formula (Ia). Reaction scheme (2) comprises step (a) in which an appropriately substituted aniline is reacted with 3-phenyl-2-propenoyl chloride, in the presence of a suitable base, such as pyridine or triethylamine, and a suitable reaction-inert solvent, such as methylene chloride or ethylene dichloride. The reaction may conveniently be carried out at a low temperature, e.g. 5°C. In a next step (b) the adduct obtained in step (a) is cyclisized in the presence of AlCl₃ and chlorobenzene. In a next step (c), the product obtained in (b) is reacted with phosphoryl chloride (POCl₃). The reaction may conveniently be carried out at a temperature ranging between room temperature and reflux temperature. In a next step (d-1), a specific R²-group, wherein R² is for example a C₁₋₆alkyloxy radical is introduced by reacting the intermediate compound obtained in step (c) with ⁻O-C₁₋₆alkyl in the presence of a suitable solvent, such as for example HO-C₁₋₆alkyl. The intermediate obtained in step (c) can also be converted into an intermediate wherein R² is for example a C₁₋₆alkylthio radical by reaction with S=C(NH₂)₂ in the presence of a suitable solvent, such as for example an alcohol, e.g. ethanol, or an alcohol/water mixture, optionally in the presence of a suitable base, such as for example KOH, (see step (d-2)) followed by reaction with C₁₋₆alkyl-I in the presence of a suitable base, such as for example K₂CO₃ and a suitable solvent, such as for example 2-propanone (see step (e)). The intermediate obtained in step (c) can also be converted into an intermediate wherein R² is -N(R^{2a})(alkyl) wherein R^{2a} is hydrogen or alkyl, by reaction with a suitable salt of NH(R^{2a})(alkyl) in the presence of a suitable base, such as for example potassium carbonate, and a suitable solvent, such as for example acetonitrile (step (d-3)). The intermediate obtained in step (c) can also be converted into an intermediate wherein R² is C₁₋₆alkyloxyC₁₋₆alkyloxy optionally substituted with C₁₋₆alkyloxy, said R² being represented by R^{2b} , by reaction with C₁₋₆alkyloxC₁₋₆alkylOH optionally substituted with C₁₋₆alkyloxy, in the presence of NaH and a suitable solvent, such as for example tetrahydrofuran (step (d-4)).

Intermediates of formula (IIb), in particular (IIb-1) or (IIb-2), can be prepared according to the following reaction scheme (3).

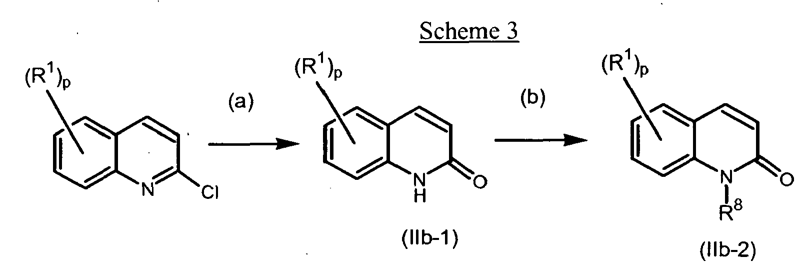

Reaction scheme (3) comprises step (a) in which the quinoline moiety (see step (c) of Scheme 2) is converted in the quinolinone moiety by reaction with a suitable acid, such as for example hydrochloric acid. In a next step (b), a R⁸ substituent is introduced by reacting the intermediate obtained in step (a) with a suitable alkylating agent, such as for example alkyliodide, e.g. methyliodide, in the presence of a suitable base, such as for example NaOH or benzyltriethylammonium chloride, a suitable solvent, such as for example tetrahydrofuran.

Intermediates of formula (IIb) wherein R⁸ and R⁹ are taken together to form the radical -CH=CH-N=, said intermediates being represented by formula (IIb-3), can be prepared according to the following reaction scheme (4).

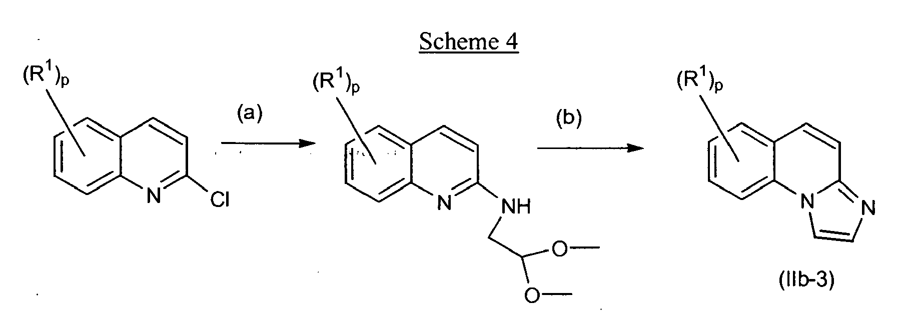

Reaction scheme (4) comprises step (a) in which the intermediate is reacted with NH₂-CH₂-CH(OCH₃)₂. In a next step (b), the fused imidazolyl moiety is formed by reaction with acetic acid in the presence of a suitable solvent, such as for example xylene.

The intermediates of formula (III) are compounds that are either commercially available or may be prepared according to conventional reaction procedures generally known in the art. For example, intermediates of formula (III) may be prepared according to the following reaction scheme (5):

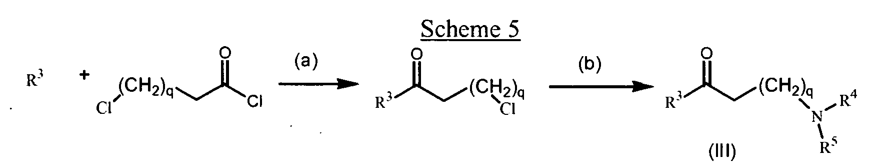

Reaction scheme (5) comprises step (a) in which R³, in particular an appropriately substituted aryl, more in particular naphthyl or an appropriately substituted phenyl, is reacted by Friedel-Craft reaction with an appropriate acylchloride such as 3-chloropropionyl chloride or 4-chlorobutyryl chloride, in the presence of a suitable Lewis acid, such as for example AlCl₃, FeCl₃, SnCl₄, TiCl₄ or ZnCl₂ and a suitable reaction-inert solvent, such as methylene chloride or ethylene dichloride. The reaction may conveniently be carried out at a temperature ranging between room temperature and reflux temperature. In a next step (b), an amino group (-NR⁴R⁵) is introduced by reacting the intermediate obtained in step (a) with a primary or secondary amine (HNR⁴R⁵)_{.}

The intermediates of formula (III) may also be prepared according to the following reaction Scheme (6) :

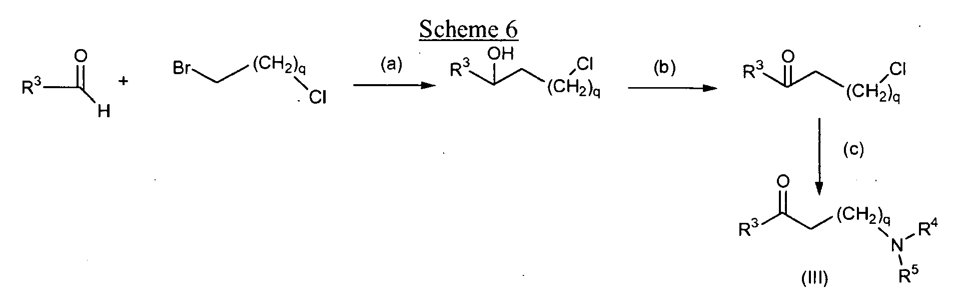

Reaction scheme (6) comprises step (a) in which R³-C(=O)-H, for instance an appropriately substituted arylcarboxaldehyde, more in particular an appropriately substituted phenyl or naphthylcarboxaldehyde, is reacted with an appropriate intermediate compound such as for example 1-bromo-4-chlorobutane, in the presence of Grignard reagent and a suitable solvent, such as for example diethyl ether, tetrahydrofuran. The reaction may conveniently be carried out at a low temperature for instance 5°C. In a next step (b), an oxidation is performed in the presence of Jones'reagent in a suitable solvent, such as for example acetone. In a next step (c), an amino group (-NR₄R₅) is introduced by reacting the intermediate compound obtained in step (b) with a primary or secondary amine HNR₄R₅ in the presence of a suitable solvent, such as for example acetonitrile, and a suitable base, such as for example K₂CO₃.

Alternatively, intermediates of formula (III) may be prepared according to the following reaction scheme (7):

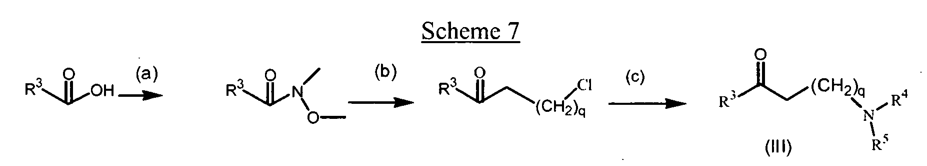

Reaction scheme (7) comprises step (a) in which for instance a suitable acid is reacted with NH(CH₃)(OCH₃) in the presence of 1,1'-carbonyldiimidazole and a suitable solvent, such as for example CH₂Cl₂. In a next step (b), the product obtained in step (a) is reacted with a suitable Grignard reagens, e.g. 4-chlorobutyl magnesium bromide, in the presence of a suitable solvent, such as for example tetrahydrofuran. In a next step (c), an amino group (-NR₄R₅) is introduced by reacting the intermediate obtained in step (b) with a primary or secondary amine HNR₄R₅ in the presence of a suitable solvent, such as for example acetonitrile, and a suitable base, such as for example K₂CO₃.

Alternatively, intermediates of formula (III) wherein q is 1, said intermediates being represented by formula (III-a), may be prepared according to the following reaction scheme (8):

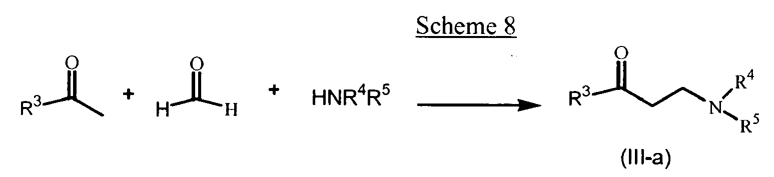

Reaction scheme (8) comprises the step in which a suitable acetyl derivative of R³ such as for example acetylcyclohexane, is reacted with paraformaldehyde and a suitable primary or secondary amine HNR⁴R⁵, preferably in its salt form, in the presence of a suitable acid, such as for example hydrochloric acid and the like, and a suitable solvent, such as for example an alcohol, e.g. ethanol.

Intermediates of formula (III) wherein R³ represents R^{3a}-CH²-CH²- (which is possible for those intermediates of formula (III) wherein R³ represents alkyl, arylalkyl, aryl-O-alkyl, aryl-alkyl-O-alkyl, Het-alkyl, Het-O-alkyl or Het-alkyl-O-alkyl and R^{3a} is the same as R³ but with 2 carbon atoms less in the alkyl chain attached to the remainder of the molecule, and wherein q represents 1, said intermediates being represented by formula (III-b), can be prepared according to the following reaction scheme (9):

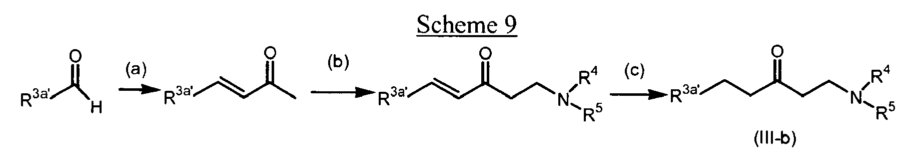

Reaction scheme (9) comprises step (a) wherein a suitable aldehyde is reacted with acetone in the presence of a suitable base, such as for example sodium hydroxide. In a next step (b), the product obtained in step (a) is reacted with a primary or secondary amine HNR⁴R⁵ in the presence of CH₂(=O), a suitable acid, such as for example hydrochloric acid and the like, and a suitable solvent, such as for example an alcohol, e.g. ethanol. In a next step (c), the product obtained in step (b) is hydrogenated (H2) in the presence of a suitable catalyst, such as for example palladium on charcoal, and a suitable solvent, such as for example water and an alcohol, e.g. ethanol.

Intermediates of formula (III) wherein R³ represents a halo substituted phenyl, may be converted into an intermediate of formula (III) wherein R³ represents phenyl substituted with aryl, by reaction with arylboronic acid in the presence of a suitable base, such as for example potassium phosphate, a suitable catalyst, such as for example palladium acetate, and a suitable ligand, such as for example 2-dicyclohexylphosphino-2',6'-dimethoxybiphenyl, in an appropriate solvent, such as for example toluene.

Intermediates of formula (III) wherein R³ represents a halo substituted phenyl, may also be converted into an intermediate of formula (III) wherein R³ represents phenyl substituted with C₂₋₆alkenyl optionally substituted with phenyl, by reaction with an appropriate C₂₋₆alkene, such as for example styrene, in the presence of a suitable base, such as for example triethylamine, a suitable catalyst, such as for example palladium acetate, and a suitable ligand, such as for example tri-o-tolylphosphine, in an appropriate solvent, such as for example DMF.

In case in the above reaction schemes, the suitable amine HNR⁴R⁵ represents substituted 2,5-diazabicyclo[2.2.1]heptyl, said amine can be prepared according to the following reaction scheme (10):

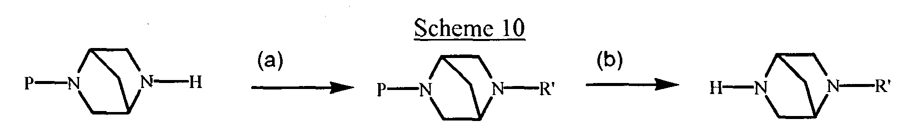

Reaction scheme (10) comprises the step of reacting an appropriately protected 2,5-diazabicyclo[2.2.1]heptyl wherein P represents for instance *tert*-butyloxycarbonyl, with an appropriate reagens of formula W-R' wherein W represents a suitable leaving group, such as for example halo, e.g. bromo and the like, and wherein R' represents the substituent to be introduced, in the presence of a suitable base, such as for example K₂CO₃, NaHCO₃ or triethylamine, a suitable phase.transfer reagent, such as for example tetra-*n*-butylammonium chloride, a suitable solvent, such as for example acetonitrile, and optionally KI to increase the speed of the reaction. In a next step (b), the protective group is removed by reaction with a suitable acid, such as for example trifluoroacetic acid in the presence of a suitable solvent, such as for example methylene chloride.

Intermediates of formula (IV-a) can be prepared according to the following reaction scheme (11):

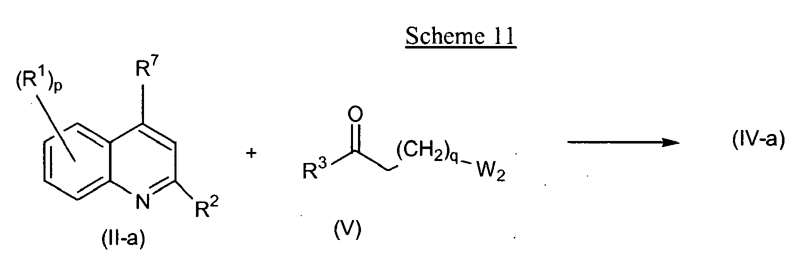

In reaction scheme (11), an intermediate of formula (II-a) is reacted with an intermediate of formula (V), for its synthesis reference is made to schemes 5, 6 and 7, in the presence of n-BuLi in a suitable solvent, such as for example tetrahydrofuran, and a suitable base, such as for example diisopropyl amine. Stirring may enhance the rate of the reaction. The reaction may conveniently be carried out at a temperature ranging between -20 and -70°C.

Intermediates of formula (IV-b) can be prepared accordingly.

The following examples illustrate the present invention without being limited thereto.

### EXPERIMENTAL PART

Of some compounds or intermediates the absolute stereochemical configuration of the stereogenic carbon atom(s) therein or the configuration at the double bond was not experimentally determined. In those cases the stereochemically isomeric form which was first isolated is designated as "A" and the second as "B", without further reference to the actual stereochemical configuration. However, said "A" and "B" isomeric forms can be unambiguously characterized by a person skilled in the art, using art-known methods such as, for example, X-ray diffraction.In some cases, when a final compound or an intermediate, indicated as a particular stereoisomer (e.g. enantiomer), is converted into another final compound/intermediate, the latter may inherit the indication of the stereochemical configuration (A, B) from the former.

Hereinafter "THF" means tetrahydrofuran.

### A. Preparation of the intermediate compounds

### Example A1

### a. Preparation of intermediate 1

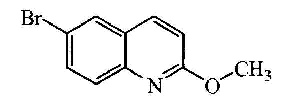

A solution of 6-bromo-2-chloroquinoline (11.56 g, 0.048 mol) and sodium methoxide 30 % in CH₃OH (45.4 ml, 0.238 mol) in CH₃OH (159 ml) was stirred at 80 °C for 16 hours. The mixture was cooled down and poured out into ice water. The organic layer was extracted with EtOAc, washed with brine, dried over MgSO₄, filtered and the solvent was evaporated. Yield: 11.38 g of **intermediate 1** (99 %).

### Example A2

### a-1. Preparation of intermediate 2a and 2b

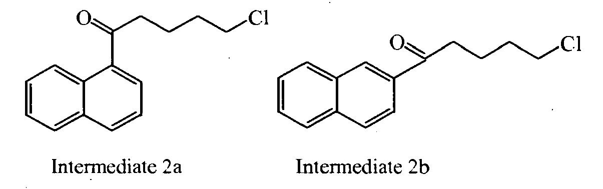

5-Chloropentanoyl chloride (0.156 mol) was added dropwise at 0 °C to a solution of AlCl₃ (0.172 mol) in CH₂Cl₂ (100 ml). A solution of naphtalene (0.156 mol) in CH₂Cl₂ (100 ml) was added dropwise. The mixture was brought to room temperature, stirred for 2 hours, poured out on ice and extracted with CH₂Cl₂. The organic layer was washed with H₂O dried (MgSO₄) filtered, and the solvent was evaporated. The residue (39.2 g) was purified by column chromatography over silica gel (eluent: CH₂Cl₂/cyclohexane 40/60; 20-45 µm). Two fractions were collected and the solvent was evaporated. Yield: 20 g of **intermediate 2a** (5-chloro-1-(1-naphthyl)pentan-1-one) and 12 g of **intermediate 2b** (5-chloro-1-(2-naphthyl)pentan-1-one).

### a-2. Preparation of intermediate 4

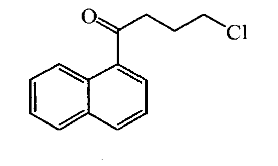

**Intermediate 4** [77972-86-2] was prepared according to the same protocol as **intermediate 2a** (A2.a-1).

### a-3. Preparation of intermediate 5

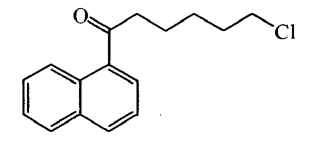

**Intermediate 5** was prepared according to the same protocol as **intermediate 2** (A2.a-1).

### b-1. Preparation of intermediate 3

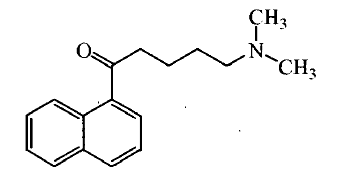

A mixture of **intermediate 2a** (0.0203 mol), *N-*methylmethanamine (0.0243 mol) and K₂CO₃ (0.0486 mol) in CH₃CN (100 ml) was stirred at 80 °C for 2 hours. Then an extra amount of *N-*methylmethanamine (1.2 equivalent) and K₂CO₃ (3.4 g) were added. The mixture was stirred at 80 °C overnight, poured out on ice and extracted with CH₂Cl₂. The organic layer was separated, dried (MgSO₄) filtered, and the solvent was evaporated. The residue (5.31 g) was purified by column chromatography over silica gel (eluent: CH₂Ch/CH₃OH/NH₄OH 95/5/0.1; 35-70 µm). The pure fractions were collected and the solvent was evaporated. Yield: 3.16 g of **intermediate 3**. (The general procedure to synthesize this kind of intermediates is also reported in WO2004/011436).

### b-2. Preparation of intermediate 6

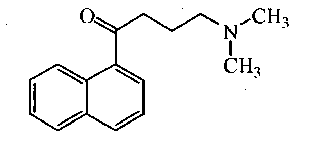

**Intermediate 6** [77252-96-1] was prepared according to the same protocol as **intermediate 3** (A2.b-1), but starting from intermediate 4.

### b-3. Preparation of intermediate 7

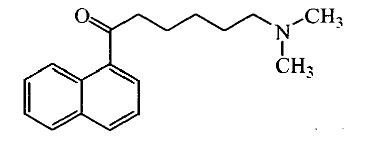

**Intermediate 7** was prepared according to the same protocol as **intermediate 3** (A2.b-1), but starting from intermediate 5.

### B. Preparation of the final compounds

### Example B1

### Preparation of compound 1

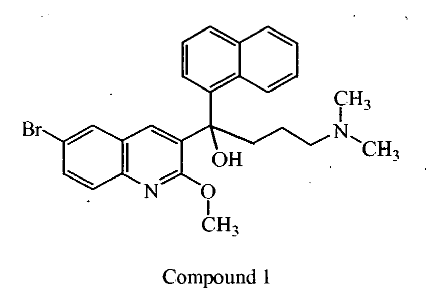

nBuLi 2.5 M in hexane (3 ml, 0.0074 mol) was added slowly at -20 °C under N₂ flow to a solution of 2,2,6,6-tetramethylpiperidine (1.16 ml, 0.0068 mol) in THF (25 ml). The mixture was stirred at -20 °C for 30 minutes, and then cooled to -70 °C. A solution of **intermediate** 1 (1.48 g, 0.0062 mol) in THF (19 ml) was added slowly. The mixture was stirred at -70 °C for 2 hours. A solution of **intermediate 6** (1.5 g, 0.0062 mol) in THF (19 ml) was added slowly. The mixture was stirred at -70 °C for 3 hours, hydrolyzed at -40 °C with water, and extracted with EtOAc. The organic layer was separated, washed with brine, dried over MgSO₄, filtered, and the solvent was evaporated. The residue was purified by column chromatography over silica gel (SiO₂ 15-40 µm, eluent: CH₂Cl₂/CH₃OH: from 98/2 to 90/10). The pure fractions were collected and the solvent was evaporated. Yield: 0.61 g of **compound 1** (20 %).

**Compound 2** was prepared according to the same protocol as compound 1, starting from **intermediate 1** and **intermediate 3**. Yield: 21 %.

**Compound 3** was prepared according to the same protocol as compound 1, starting from **intermediate 1** and **intermediate 7**. Yield: 24 %.

### Example B2

### Preparation of compound 4

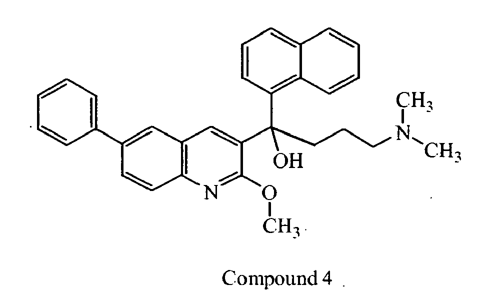

A solution of **compound 1** (0.2 g, 0.00040 mol), phenylboronic acid (0.073 g, 0.00063 mol) and tetrakis(triphenylphosphine)palladium (0.046 g, 0.00004 mol) in 1,2-dimethoxyethane (1.5 ml), CH₃OH (1 ml) and a 2 M solution of Na₂CO₃ (0.3 ml) was stirred for 10 minutes at 120 °C in the microwave. Then the mixture was cooled to room temperature and poured out into water and extracted with CH₂Cl₂. The combined organic layers were dried over MgSO₄ filtered and concentrated. The residue was purified by column chromatography over silica gel (SiO₂ 15-40 µm, eluent: CH₂Cl₂/CH₃OH: from 98/2 to 90/10). The pure fractions were collected and the solvent was evaporated. The product was crystallized from diethyl ether. Yield: 0.053 g of **compound 4** (28 %).

**Compound 5** was prepared according to the same protocol as compound 4, starting from **compound 2** and phenylboronic acid. Yield: 46 %.

**Compound 6** was prepared according to the same protocol as compound 4, starting from **compound 3** and phenylboronic acid. Yield: 56 %.

**Compound 7** was prepared according to the same protocol as compound 4, starting from **compound 1** and 2-methoxyphenylboronic acid. Yield: 26 %.

**Compound 8** was prepared according to the same protocol as compound 4, starting from **compound 2** and 2-methoxyphenylboronic acid. Yield: 34 %.

**Compound 9** was prepared according to the same protocol as compound 4, starting from **compound 3** and 2-methoxyphenylboronic acid. Yield: 90 %.

**Compound 10** was prepared according to the same protocol as compound 4, starting from **compound 1** and 3-furanylboronic acid. Yield: 45 %.

**Compound 11** was prepared according to the same protocol as compound 4, starting from **compound 2** and 3-furanylboronic acid. Yield: 100 %.

**Compound 12** was prepared according to the same protocol as compound 4, starting from **compound 3** and 3-furanylboronic acid. Yield: 42 %.

**Compound 13** was prepared according to the same protocol as compound 4, starting from **compound 1** and 2-thienylboronic acid. Yield: 43 %.

**Compound 14** was prepared according to the same protocol as compound 4, starting from **compound 2** and 2-thienylboronic acid. Yield: 39 %.

**Compound 15** was prepared according to the same protocol as compound 4, starting from **compound 3** and 2-thienylboronic acid. Yield: 24 %.

**Compound 16** was prepared according to the same protocol as compound 4, starting from **compound 1** and (3-pyridinyl)boronic acid. Yield: 41 %.

**Compound 17** was prepared according to the same protocol as compound 4, starting from **compound 2** and (3-pyridinyl)boronic acid. Yield: 37 %.

**Compound 18** was prepared according to the same protocol as compound 4, starting from **compound 3** and (3-pyridinyl)boronic acid. Yield: 50 %.

**Compound 19** was prepared according to the same protocol as compound 4, starting from **compound 1** and 2-benzofuranyl-boronic acid. Yield: 72 %.

**Compound 20** was prepared according to the same protocol as compound 4, starting from **compound 3** and 2-benzofurariyl-boronic acid. Yield: 49 %.

Table 1 lists compounds of formula (Ia) according to the present invention and which were prepared according to one of the above procedures (Ex. nr.).

**Table 1 :**

| | | | |
|---|---|---|---|
| 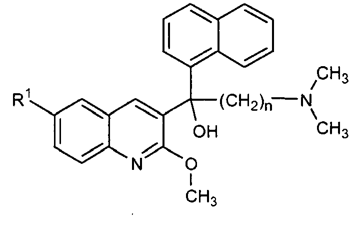 | | | |

| Comp. nr. | Ex. nr. | R¹ | n |
|---|---|---|---|
| 1 | B1 | --Br | 3 |
| 2 | B1 | --Br | 4 |
| 3 | B1 | --Br | 5 |
| 4 | B2 | 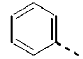 | 3 |
| 5 | B2 | 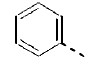 | 4 |
| 6 | B2 | 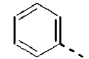 | 5 |
| 7 | B2 | 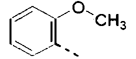 | 3 |
| 8 | B2 | 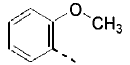 | 4 |
| 9 | B2 | 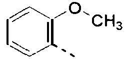 | 5 |
| 10 | B2 | 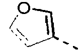 | 3 |
| 11 | B2 | 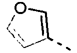 | 4 |
| 12 | B2 | 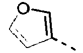 | 5 |
| 13 | B2 | 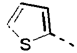 | 3 |
| 14 | B2 | 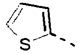 | 4 |
| 15 | B2 | 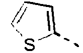 | 5 |
| 16 | B2 | 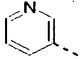 | 3 |
| 17 | B2 | 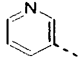 | 4 |
| 18 | B2 | 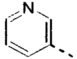 | 5 |
| 19 | B2 | 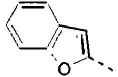 | 3 |
| 20 | B2 | 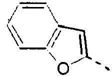 | 5 |

### C. Analytical methods

### LCMS

The mass of some compounds was recorded with LCMS (liquid chromatography mass spectrometry). The methods used are described below.

### General procedure

The HPLC measurement was performed using an Agilent 1100 series liquid chromatography system comprising a binary pump with degasser, an autosampler, a column oven, a UV detector and a column as specified in the respective methods below. Flow from the column was split to a MS spectrometer. The MS detector was configured with an electrospray ionization source. The capillary voltage was 3 kV, the quadrupole temperature was maintained at 100 °C and the desolvation temperature was 300 °C. Nitrogen was used as the nebulizer gas. Data acquisition was performed with an Agilent Chemstation data system.

### Method 1

In addition to the general procedure: Reversed phase HPLC was carried out on a YMC-Pack ODS-AQ C 18 column (4.6 x 50 mm) with a flow rate of 2.6 ml/min. A gradient run was used from 95 % water and 5 % acetonitrile to 95 % acetonitrile in 7.30 minutes and was hold for 1.20 minutes. Mass spectra were acquired by scanning from 100 to 1000. Injection volume was 10 µl. Column temperature was 35 °C.

### Method 2

In addition to the general procedure: Reversed phase HPLC was carried out on a YMC-Pack ODS-AQ C18 column (4.6 x 50 mm) with a flow rate of 2.6 ml/min. A gradient run was used from 88 % water and 12 % acetonitrile to 88 % acetonitrile in 3.40 minutes and was hold for 1.20 minutes. Mass spectra were acquired by scanning from 110 to 1000. Injection volume was 10 µl. Column temperature was 35 °C.

When a compound is a mixture of isomers which give different peaks in the LCMS method , only the retention time of the main component is given in the LCMS table.

**Table 2 : LCMS: (MH⁺), protonated molecular ion (of the free base), and Rₜ, retention time in minutes.**

| **Compound No** | **LCMS method** | **(MH⁺)** | **Rₜ (min)** |
|---|---|---|---|
| 1 | 1 | 479 | 3.49 |
| 2 | 1 | 493 | 3.65 |
| 3 | 2 | 507 | 2.14 |
| 4 | 1 | 477 | 3.69 |
| 5 | 2 | 491 | 2.30 |
| 6 | 2 | 505 | 2.33 |
| 7 | 1 | 507 | 3.78 |
| 8 | 2 | 521 | 2.24 |
| 9 | 2 | 535 | 2.25 |
| 10 | 1 | 467 | 3.49 |
| 11 | 1 | 481 | 3.7 |
| 12 | 1 | 495 | 3.43 |
| 13 | 1 | 483 | 3.76 |
| 14 | 2 | 497 | 2.27 |
| 15 | 2 | 511 | 2.34 |
| 16 | 1 | 478 | 2.6 |
| 17 | 1 | 492 | 2.65 |
| 18 | 1 | 506 | 2.66 |
| 19 | 1 | 517 | 4.17 |
| 20 | 1 | 545 | 4.36 |

### D. Pharmacological examples

### D.1. In-vitro method for testing compounds against M. tuberculosis.

Flat-bottom, sterile 96-well plastic microtiter plates were filled with 100 µl of Middlebrook (1x) broth medium. Subsequently, stock solutions (10 x final test concentration) of compounds were added in 25 µl volumes to a series of duplicate wells in column 2 so as to allow evaluation of their effects on bacterial growth. Serial five-fold dilutions were made directly in the microtiter plates from column 2 to 11 using a customised robot system (Zymark Corp., Hopkinton, MA). Pipette tips were changed after every 3 dilutions to minimize pipetting errors with high hydrophobic compounds. Untreated control samples with (column 1) and without (column 12) inoculum were included in each microtiter plate. Approximately 5000 CFU per well of Mycobacterium tuberculosis (strain H37RV), in a volume of 100 µl in Middlebrook (1x) broth medium, was added to the rows A to H, except column 12. The same volume of broth medium without inoculum was added to column 12 in row A to H. The cultures were incubated at 37°C for 7 days in a humidified atmosphere (incubator with open air valve and continuous ventilation). One day before the end of incubation, 6 days after inoculation, Resazurin (1:5) was added to all wells in a volume of 20 µl and plates were incubated for another 24 hours at 37°C. On day 7 the bacterial growth was quantitated fluorometrically.

The fluorescence was read in a computer-controlled fluorometer (Spectramax Gemini EM, Molecular Devices) at an excitation wavelength of 530 nm and an emission wavelength of 590 nm. The percentage growth inhibition achieved by the compounds can be calculated according to standard methods and expressed as IC₉₀ (µg/ml) which defines the 90 % inhibitory concentration for bacterial growth.

### D.2. In-vitro method for testing compounds for anti-bacterial activity against strain M. Smegmatis ATCC607.

Flat-bottom, sterile 96-well plastic microtiter plates were filled with 180 µl of sterile deionized water, supplemented with 0.25 % BSA. Subsequently, stock solutions (7.8 x final test concentration) of compounds were added in 45 µl volumes to a series of duplicate wells in column 2 so as to allow evaluation of their effects on bacterial growth. Serial five-fold dilutions (45 µl in 180 µl) were made directly in the microtiter plates from column 2 to 11 using a customised robot system (Zymark Corp., Hopkinton, MA). Pipette tips were changed after every 3 dilutions to minimize pipetting errors with high hydrophobic compounds. Untreated control samples with (column 1) and without (column 12) inoculum were included in each microtiter plate. Approximately 250 CFU per well of bacteria inoculum, in a volume of 100 µl in 2.8x Mueller-Hinton broth medium, was added to the rows A to H, except column 12. The same volume of broth medium without inoculum was added to column 12 in row A to H. The cultures were incubated at 37°C for 48 hours in a humidified 5% CO₂ atmosphere (incubator with open air valve and continuous ventilation). At the end of incubation, two days after inoculation, the bacterial growth was quantitated fluorometrically. Therefore Alamar Blue (10x) was added to all wells in a volume of 20 µl and plates were incubated for another 2 hours at 50°C.

The fluorescence was read in a computer-controlled fluorometer (Cytofluor, Biosearch) at an excitation wavelength of 530 nm and an emission wavelength of 590 nm (gain 30). The percentage growth inhibition achieved by the compounds was calculated according to standard methods and expressed as IC₉₀ (µg/ml) which defines the 90 % inhibitory concentration for bacterial growth. See Table 3.

### D.3. In-vitro method for testing compounds for anti-bacterial activity against various non-mycobacterial strains

### Preparation of bacterial suspensions for susceptibility testing:

The bacteria used in this study were grown overnight in flasks containing 100 ml Mueller-Hinton Broth (Becton Dickinson - cat. no. 275730) in sterile de-ionized water, with shaking, at 37 °C. Stocks (0.5 ml/tube) were stored at -70 °C until use. Bacteria titrations were performed in microtiter plates to detect the TCID₅₀, in which the TCID50 represents the dilution that gives rise to bacterial growth in 50 % of inoculated cultures.
In general, an inoculum level of approximately 100 TCID₅₀ was used for susceptibility testing.

### Anti bacterial Susceptibility testing: IC₉₀ determination

### Microtitre plate assay

Flat-bottom, sterile 96-well plastic microtiter plates were filled with 180 µl of sterile deionized water, supplemented with 0.25 % BSA. Subsequently, stock solutions (7.8 x final test concentration) of compounds were added in 45 µl volumes in column 2. Serial five-fold dilutions (45 µl in 180 µl) were made directly in the microtiter plates from column 2 to reach column 11. Untreated control samples with (column 1) and without (column 12) inoculum were included in each microtiter plate. Depending on the bacteria type, approximately 10 to 60 CFU per well of bacteria inoculum (100 TCID50), in a volume of 100 µl in 2.8x Mueller-Hinton broth medium, was added to the rows A to H, except column 12. The same volume of broth medium without inoculum was added to column 12 in row A to H. The cultures were incubated at 37°C for 24 hours under a normal atmosphere (incubator with open air valve and continuous ventilation). At the end of incubation, one day after inoculation, the bacterial growth was quantitated fluorometrically. Therefore resazurin (0.6 mg/ml) was added in a volume of 20 µl to all wells 3 hours after inoculation, and the plates were re-incubated overnight. A change in colour from blue to pink indicated the growth of bacteria.
The fluorescence was read in a computer-controlled fluorometer (Cytofluor Biosearch) at an excitation wavelength of 530 nm and an emission wavelength of 590 nm. The % growth inhibition achieved by the compounds was calculated according to standard methods. The IC₉₀ (expressed in µg/ml) was defined as the 90 % inhibitory concentration for bacterial growth. The results are shown in Table 3.

### Agar dilution method.

MIC₉₉ values (the minimal concentration for obtaining 99 % inhibition of bacterial growth) can be determined by performing the standard Agar dilution method according to NCCLS standards* wherein the media used includes Mueller-Hinton agar. * Clinical laboratory standard institute. 2005. Methods for dilution Antimicrobial susceptibility tests for bacteria that grows Aerobically: approved standard -sixth edition

### Time kill assays

Bactericidal or bacteriostatic activity of the compounds may be determined in a time kill assay using the broth microdilution method *. In a time kill assay on *Staphylococcus aureus* and methicillin resistant *S. aureus* (MRSA), the starting inoculum of *S. aurues* and MRSA is 10⁶ CFU / ml in Muller Hinton broth. The antibacterial compounds are used at the concentration of 0.1 to 10 times the MIC (i.e. IC₉₀ as determined in microtitre plate assay). Wells receiving no antibacterial agent constitute the culture growth control. The plates containing the microorganism and the test compounds are incubated at 37 °C. After 0, 4, 24, and 48 hrs of incubation samples are removed for determination of viable counts by serial dilution (10⁻¹ to 10⁻⁶) in sterile PBS and plating (200 µl) on Mueller Hinton agar. The plates are incubated at 37 °C for 24 hrs and the number of colonies are determined. Killing curves can be constructed by plotting the log₁₀CFU per ml versus time. A bactericidal effect is commonly defined as 3-log₁₀ decrease in number of CFU per ml as compared to untreated inoculum. The potential carryover effect of the drugs is removed by serial dilutions and counting the colonies at highest dilution used for plating.
* Zurenko,G.E. et al. In vitro activities of U-100592 and U-100766, novel oxazolidinone antibacterial agents. Antimicrob. Agents Chenzother. 40, 839-845 (1996).

### Determination of cellular A TP levels

In order to analyse the change in the total cellular ATP concentration (using ATP bioluminescence Kit, Roche), assays are carried out by growing a culture of *S. aureus* (ATCC29213) stock in 100 ml Mueller Hinton flasks and incubate in a shaker-incubator for 24 hrs at 37 °C (300 rpm). Measure OD₄₀₅ nm and calculate the CFU/ml. Dilute the cultures to 1 x 10⁶ CFU/ml (final concentration for ATP measurement: 1 x 10⁵ CFU/100 µl per well) and add test compound at 0.1 to 10 times the MIC (i.e. IC₉₀ as determined in microtitre plate assay). Incubate these tubes for 0, 30 and 60 minutes at 300 rpm and 37°C. Use 0.6 ml bacterial suspension from the snap-cap tubes and add to a new 2 ml eppendorf tubes. Add 0.6 ml cell lysis reagent (Roche kit), vortex at max speed and incubate for 5 minutes at room temperature. Cool on ice. Let the luminometer warm up to 30°C (Luminoskan Ascent Labsystems with injector). Fill one column (= 6 wells) with 100 µl of the same sample. Add 100 µl Luciferase reagent to each well by using the injector system. Measure the luminescence for 1 sec.

**Table 3: IC₉₀ values (µg/ml).**

| | **IC90 (µg/ml)** | | |
|---|---|---|---|
| **Comp. No.** | **STA 29213** | **SPN 6305** | **MSM ATCC60**7 |
| 1 | 7.60 | 8.53 | 1.91 |
| 2 | 39.20 | 9.85 | 7.82 |
| 3 | 8.04 | 10.13 | 11.36 |
| 4 | 1.51 | 1.51 | 1.51 |
| 5 | 38.97 | 49.07 | 7.78 |
| 6 | 8.00 | 10.07 | 6.35 |
| 7 | 1.60 | 1.8 | 1.60 |
| 8 | 8.25 | 10.39 | 6.55 |
| 9 | 8.47 | 8.47 | 8.47 |
| 10 | 1.66 | 3.30 | 1.48 |
| 11 | 7.62 | 9.59 | 7.62 |
| 12 | 1.56 | 1.76 | 1.56 |
| 13 | 1.53 | 1.71 | 1.53 |
| 14 | 39.45 | 49.67 | 15.71 |
| 15 | 51.07 | 51.07 | 45.52 |
| 16 | 7.57 | 7.57 | 1.51 |
| 17 | 49.16 | 49.16 | 49.16 |
| 18 | 8.01 | 8.99 | 8.01 |
| 19 | 3.66 | 8.19 | 3.26 |
| 20 | 1.72 | 1.72 | 1.72 |

STA 29213 means *Staphylococcus aureus* (ATCC29213); SPN 6305 means *Streptococcus pneumoniae* (ATCC6305); MSM 607 means *M. Smegmatis* (ATCC607); ATCC means American type tissue culture;

## Claims

1. A compound of formula (Ia) or (Ib) 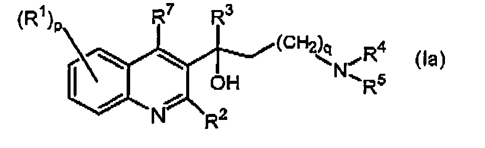 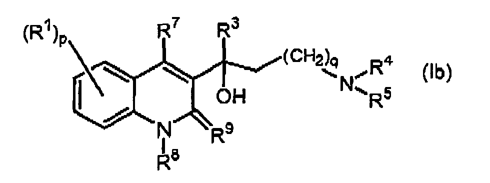 including any stereochemically isomeric form thereof, wherein
p is an integer equal to 1, 2, 3 or 4;
q is an integer equal to zero, 1, 2, 3 or 4;
R¹ is hydrogen, cyano, formyl, carboxyl, halo, alkyl, C₂₋₆alkenyl, C₂₋₆alkynyl, haloalkyl, hydroxy, alkyloxy, alkylthio, alkylthioalkyl -C=N-OR¹¹, amino, mono or di(alkyl)amino, aminoalkyl, mono or di(alkyl)aminoalkyl, alkylcarbonylaminoalkyl, aminocarbonyl, mono or di(alkyl)aminocarbonyl, arylalkyl, arylcarbonyl, R^{5a}R^{4a}Nalkyl, di(aryl)alkyl, aryl, R^{5a}R^{4a}N-, R^{5a}R^{4a}N-C(=O)-, or Het;
R² is hydrogen, alkyloxy, aryl, aryloxy, hydroxy, mercapto, alkyloxyalkyloxy, alkylthio, mono or di(alkyl)amino, pyrrolidino or a radical of formula 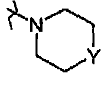 wherein Y is CH₂, O, S, NH or *N-*alkyl;
R³ is alkyl, arylalkyl, aryl-O-alkyl, aryl-alkyl-O-alkyl, aryl, aryl-aryl, Het, Het-alkyl, Het-O-alkyl, Het-alkyl-O-alkyl or 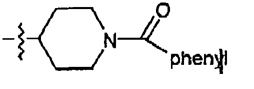
R⁴ and R⁵ each independently is hydrogen; alkyl; alkyloxyalkyl; arylalkyl; Het-alkyl; mono- or dialkylaminoalkyl; bicyclo[2.2.1]heptyl; Het; aryl; or-C(=NH)-NH₂; or
R⁴ and R⁵ together with the nitrogen atom to which they are attached form a radical selected from the group consisting of pyrrolidino, piperidin piperazino, morpholino, 4-thiomorpholino, 1,1-dioxide-thiomorpholinyl, azetidinyl, 2,3-dihydroisoindol-1-yl, thiazolidin-3-yl, 1,2,3,6- tetrahydropyridyl, hexahydro-1*H-*azepinyl, hexahydro-1*H*-1,4- diazepinyl, hexahydro-1,4-oxazepinyl, 1,2,3,4-tetrahydroisoquinolin-2- yl, 2,5-diazabicyclo[2.2,1]heptyl, pyrrolinyl, pyrrolyl, imidazolidinyl, pyrazolidinyl, 2-imidazolinyl, 2-pyrazolinyl, imidazolyl, pyrazolyl, triazolyl, pyridinyl, pyridazinyl, pyrimidinyl, pyrazinyle and triazinyl, each radical optionally substituted with 1, 2, 3 or 4 substituents, each substituent independently selected from alkyl, haloalkyl, alkylcarbonyl, halo, arylalkyl, hydroxy, alkyloxy, amino, mono- or dialkylamino, aminoalkyl, mono- or dialkylaminoalkyl, alkylthio, alkylthioalkyl, aryl, pyridyl, pyrimidinyl, piperidinyl optionally substituted with alkyl or pyrrolidinyl optionally substituted with arylalkyl;
R^{4a} and R^{5a} together with the nitrogen atom to which they are attached form a radical selected from the group consisting of pyrrolidino, piperidino, piperazino, morpholino, 4-thiomorpholino, 2,3-dihydroisoindol-1-yl, thiazolidin-3-yl, 1,2,3,6-tetrahydropyridyl, hexahydro-1*H-*azepinyl, hexahydro-1*H*-1,4-diazepinyl, hexahydro-1,4-oxazepinyl, 1,2,3,4- tetrahydroisoquinolin-2-yl, pyrrolinyl, pyrrolyl, imidazolidinyl, pyrazolidinyl, 2-imidazolinyl, 2-pyrazolinyl, imidazolyl, pyrazolyl triazolyl, pyridinyl, pyridazinyl, pyrimidinyl, pyrazinyl and triazinyl, each radical optionally substituted with 1, 2, 3 or 4 substituents, each substituent independently selected from alkyl, haloalkyl, halo, arylalkyl, hydroxy, alkyloxy, amino, mono- or dialkylamino, alkylthio, alkylthioalkyl, aryl, pyridyl or pyrimidinyl;
R⁷ is hydrogen, halo, alkyl, aryl or Het;
R⁸ is hydrogen or alkyl;
R⁹ is oxo; or
R⁸ and R⁹ together form the radical -CH=CH-N=;
R¹¹ is hydrogen or alkyl;
aryl is a homocycle selected from phenyl, naphthyl, acenaphthyl or tetrahydronaphthyl, each being optionally substituted with 1,2 or 3 substituents, each substituent being independently selected from hydroxy, halo, cyano, nitro, amino, mono- or dialkylamino, alkyl, C₂₋₆alkenyl optionally substituted with phenyl, haloalkyl, alkyloxy, haloalkyloxy, carboxyl, alkyloxycarbonyl, aminocarbonyl, morpholinyl or mono- or dialkylaminocarbonyl;
Het is a monocyclic heterocycle selected from *N-*phenoxypiperidinyl, piperidinyl, pyrrolyl, pyrazolyl, imidazolyl, furanyl, thienyl, oxazolyl, isoxazolyl, thiazolyl, isothiazolyl, pyridinyl, pyrimidinyl, pyrazinyl or pyridazinyl; or a bicyclic heterocycle selected from quinolinyl, quinoxalinyl, indolyl, benzimidazolyl, benzoxazolyl, benzisoxazolyl, benzothiazolyl, benzisothiazolyl, benzofuranyl, benzothienyl, 2,3-dihydxobenzo[1,4]dioxinyl orbenzo[1,3]dioxolyl; each monocyclic and bicyclic heterocycle being optionally substituted with 1, 2 or 3 substituents, each substituent independently selected from halo, hydroxy, alkyl or alkyloxy;
a *N-*oxide thereof, a pharmaceutically acceptable salt thereof or a solvateas thereof.

2. A compound according to claim 1 wherein
R³ is alkyl, arylalkyl, aryl-O-alkyl, aryl-alkyl-O-alkyl, aryl, Het, Het-alkyl, Het-O-alkyl, Het-alkyl-O-alkyl or 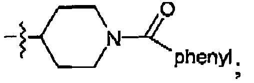
R⁴ and R⁵ each independently is hydrogen; alkyl; alkyloxyalkyl; arylalkyl; Het-alkyl; mono- or dialkylaminoalkyl; Het; aryl; or -C(=NH)-NH₂; or
R⁴ and R⁵ together with the nitrogen atom to which they are attached form a radical selected from the group consisting of pyrrolidino, piperidino, piperazino, morpholino, 4-thiomorpholino, 2,3-dihydroisoindol-1-yl, thiazolidin-3-yl, 1,2,3,6-tetrahydropytidyl, hexahydro-1*H*-azepinyl, hexahydro-1*H*-1,4-diazepinyl, hexahydro-1,4-oxazepinyl, 1,2,3,4- tetrahydroisoquinolin-2-yl, 2,5-diazabicyclo[2.2.1]heptyl, pyrrolinyl, pyrrolyl, imidazolidinyl, pyrazolidinyl, 2-imidazolinyl, 2-pyrazolinyl, imidazolyl, pyrazolyl, triazolyl, pyridinyl, pyridazinyl, pyrimidinyl, pyrazinyl and triazinyl, each radical optionally substituted with 1,2, 3 or 4 substituents, each substituent independently selected from alkyl, haloalkyl, alkylcarbonyl, halo, arylalkyl, hydroxy, alkyloxy, amino, mono- or dialkylamino, alkylthio, alkylthioalkyl, aryl, pyridyl, pyrimidinyl, piperidinyl or pyrrolidinyl optionally substituted with arylalkyl;
aryl is a homocycle selected from phenyl, naphthyl, acenaphthyl or tetrahydronaphthyl, each being optionally substituted with 1, 2 or 3 substituents, each substituent being independently selected from hydroxy, halo, cyano, nitro, amino, mono- or dialkylamino, alkyl, haloalkyl, alkyloxy, haloalkyloxy, carboxyl, alkyloxycarbonyl, aminocarbonyl, morpholinyl or mono- or dialkylaminocarbonyl.

3. A compound according to claim 1 or 2 wherein alkyl represents C₁₋₆alkyl.

4. A compound according to any one of the preceding claims wherein R¹ is halo, Het or aryl.

5. A compound according to claim 4 wherein R¹ is halo.

6. A compound according to any one of the preceding claims wherein p is equal to 1.

7. A compound according to any one of the preceding claims wherein R² is C₁₋₆alkyloxy.

8. A compound according to any one of the preceding claims wherein R³ is aryl.

9. A compound according to any one of the preceding claims wherein q is equal to 2, 3 or 4.

10. A compound according to any one of the preceding claims wherein R⁴ and R⁵ represent C₁₋₆alkyl.

11. A compound according to any one of the preceding claims wherein R⁷ is hydrogen.

12. A compound according to any one of the preceding claims wherein the compound is a compound of formula (Ia).

13. A compound according to claim 12 wherein R¹ is halo; R² is C₁₋₆alkyloxy; R⁸ is aryl; R⁴ and R⁵ are C₁₋₆alkyl; R⁷ is hydrogen; q is 2, 3 or 4; and p is 1.

14. A compound according to any one of the preceding claims for use as a medicine.

15. A compound according to any one of claims 1 to 13 for use as a medicine for the treatment of a bacterial infection including a mycobacterial infection.

16. A pharmaceutical composition comprising a pharmaceutically acceptable carrier and, as active ingredient, a therapeutically effective amount of a compound as defined in any one of claims 1 to 13.

17. A compound according to claim 15 wherein the bacterial infection is an infection with a gram-positive bacterium.

18. A compound according to claim 15 wherein the gram-positive bacterium is Streptococcus pneumoniae or Staphylococcus aureus.

19. A combination of (a) a compound according to any one of claims 1 to 13, and (b) one or more other antibacterial agents.

20. A product containing (a) a compound according to any one of claims 1 to 13, and (b) one or more other antibacterial agents, as a combined preparation for simultaneous, separate or sequential use in the treatment of a bacterial infection.

21. A compound according to claim 18 wherein the gram-positive bacterium is methicillin resistant *Staphylococcus aureus.*

## Patentansprüche

1. Verbindung der Formel (Ia) oder (Ib) 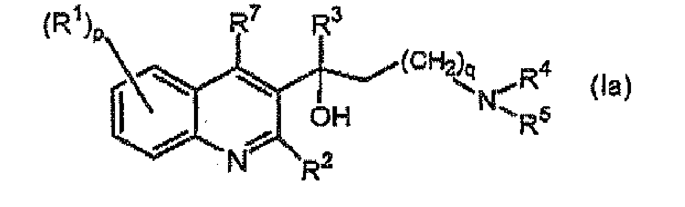 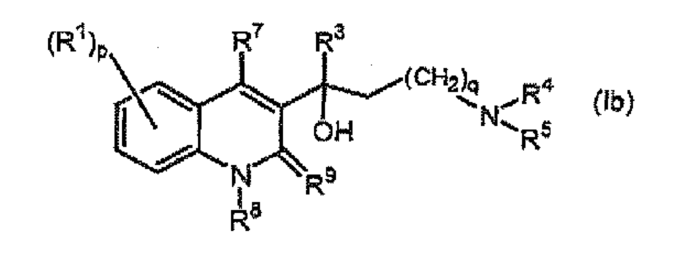 einschließlich beliebiger stereochemisch isomerer Formen davon, worin
p für eine ganze Zahl mit einem Wert von 1, 2, 3 oder 4 steht;
q für eine ganze Zahl mit einem Wert von null, 1, 2, 3 oder 4 steht;
R¹ für Wasserstoff, Cyano, Formyl, Carboxyl, Halogen, Alkyl, C₂₋₆-Alkenyl, C₂₋₆-Alkinyl, Halogenalkyl, Hydroxy, Alkyloxy, Alkylthio, Alkylthioalkyl, -C=N-OR¹¹, Amino, Mono- oder Di- (alkyl)amino, Aminoalkyl, Mono- oder Di(alkyl)- aminoalkyl, Alkylcarbonylaminoalkyl, Amino- carbonyl, Mono- oder Di(alkyl)aminocarbonyl, Arylalkyl, Arylcarbonyl, R^{5a}R^{4a}N-Alkyl, Di(aryl)- alkyl, Aryl, R^{5a}R^{4a}N-, R^{5a}R^{4a}N₋C (=O) - oder Het steht;
R² für Wasserstoff, Alkyloxy, Aryl, Aryloxy, Hydroxy, Mercapto, Alkyloxyalkyloxy, Alkylthio, Mono- oder Di(alkyl)amino, Pyrrolidino oder einen Rest der Formel 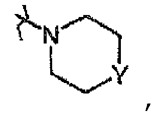 worin Y CH₂, O, S, NH oder *N-*Alkyl bedeutet, steht;
R³ für Alkyl, Arylalkyl, Aryl-O-alkyl, Arylalkyl- O-alkyl, Aryl, Arylaryl, Het, Het-Alkyl, Het-O- Alkyl, Het-Alkyl-O-alkyl oder 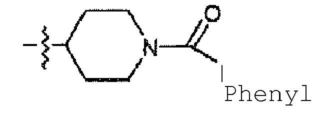 steht;
R⁴ und R⁵ jeweils unabhängig voneinander für Wasserstoff; Alkyl; Alkyloxyalkyl; Arylalkyl; Het-Alkyl; Mono- oder Dialkylaminoalkyl; Bicyclo[2.2.1]heptyl; Het; Aryl oder -C(=NH)-NH₂ stehen; oder
R⁴ und R⁵ gemeinsam mit dem Stickstoffatom, an das sie gebunden sind, einen Rest aus der Gruppe bestehend aus Pyrrolidino, Piperidino, Piperazino, Morpholino, 4-Thiomorpholino, Thiomorpholinyl-1,1-dioxid, Azetidinyl, 2,3- Dihydroisoindol-1-yl, Thiazolidin-3-yl, 1,2,3,6-Tetrahydropyridyl, Hexahydro-1*H-* azepinyl, Hexahydro-1*H-*1,4-diazepinyl, Hexahydro-1,4-oxazepinyl, 1,2,3,4- Tetrahydroisochinolin-2-yl, 2,5-Diazabicyclo- [2.2.1]heptyl, Pyrrolinyl, Pyrrolyl, Imidazolidinyl, Pyrazolidinyl, 2-Imidazolinyl, 2-Pyrazolinyl, Imidazolyl, Pyrazolyl, Triazolyl, Pyridinyl, Pyridazinyl, Pyrimidinyl, Pyrazinyl und Triazinyl bilden, wobei jeder Rest gegebenenfalls durch 1, 2, 3 oder 4 Substituenten substituiert ist, wobei jeder Substituent unabhängig unter Alkyl, Halogen- alkyl, Alkylcarbonyl, Halogen, Arylalkyl, Hydroxy, Alkyloxy, Amino, Mono- oder Dialkyl- amino, Aminoalkyl, Mono- oder Dialkylamino- alkyl, Alkylthio, Alkylthioalkyl, Aryl, Pyridyl, Pyrimidinyl, Piperidinyl, das gegebenenfalls durch Alkyl substituiert ist, oder Pyrrolidinyl, das gegebenenfalls durch Arylalkyl substituiert ist, ausgewählt ist;
R^{4a} und R^{5a} gemeinsam mit dem Stickstoffatom, an das sie gebunden sind, einen Rest aus der Gruppe bestehend aus Pyrrolidino, Piperidino, Piperazino, Morpholino, 4-Thiomorpholino, 2,3- Dihydroisoindol-1-yl, Thiazolidin-3-yl, 1,2,3,6-Tetrahydropyridyl, Hexahydro-1*H-* azepinyl, Hexahydro-1*H-*1,4-diazepinyl, Hexa- hydro-1,4-oxazepinyl, 1,2,3,4-Tetrahydroiso- chinolin-2-yl, Pyrrolinyl, Pyrrolyl, Imidazolidinyl, Pyrazolidinyl, 2-Imidazolinyl, 2-Pyrazolinyl, Imidazolyl, Pyrazolyl, Triazolyl, Pyridinyl, Pyridazinyl, Pyrimidinyl, Pyrazinyl und Triazinyl bilden, wobei jeder Rest gegebenenfalls durch 1, 2, 3 oder 4 Substituenten substituiert ist, wobei jeder Substituent unabhängig unter Alkyl, Halogen- alkyl, Halogen, Arylalkyl, Hydroxy, Alkyloxy, Amino, Mono- oder Dialkylamino, Alkylthio, Alkylthioalkyl, Aryl, Pyridyl oder Pyrimidinyl ausgewählt ist;
R⁷ für Wasserstoff, Halogen, Alkyl, Aryl oder Het steht;
R⁸ für Wasserstoff oder Alkyl steht;
R⁹ für Oxo steht oder
R⁸ und R⁹ gemeinsam den Rest -CH=CH-N= bilden; R¹¹ für Wasserstoff oder Alkyl steht;
Aryl für einen Homocyclus steht, der unter Phenyl, Naphthyl, Acenaphthyl oder Tetrahydronaphthyl ausgewählt ist, wobei jeder dieser Cyclen gegebenenfalls durch 1, 2 oder 3 Substituenten substituiert ist, wobei jeder Substituent unabhängig unter Hydroxy, Halogen, Cyano, Nitro, Amino, Mono- oder Dialkylamino, Alkyl, C₂₋₆-Alkenyl, das gegebenenfalls durch Phenyl substituiert ist, Halogenalkyl, Alkyloxy, Halogenalkyloxy, Carboxyl, Alkyloxycarbonyl, Aminocarbonyl, Morpholinyl oder Mono- oder Dialkylaminocarbonyl ausgewählt ist;
Het für einen monocyclischen Heterocyclus, der unter *N-*Phenoxypiperidinyl, Piperidinyl, Pyrrolyl, Pyrazolyl, Imidazolyl, Furanyl, Thienyl, Oxazolyl, Isoxazolyl, Thiazolyl, Isothiazolyl, Pyridinyl, Pyrimidinyl, Pyrazinyl oder Pyridazinyl ausgewählt ist; oder einen bicyclischen Heterocyclus, der unter Chinolinyl, Chinoxalinyl, Indolyl, Benz- imidazolyl, Benzoxazolyl, Benzisoxazolyl, Benzothiazolyl, Benzisothiazolyl, Benzofuranyl, Benzothienyl, 2,3-Dihydrobenzo[1,4]dioxinyl oder Benzo[1,3]dioxolyl ausgewählt ist, steht; wobei jeder monocyclische und bicyclische Heterocyclus gegebenenfalls durch 1, 2 oder 3 Substituenten substituiert ist, wobei jeder Substituent unabhängig unter Halogen, Hydroxy, Alkyl oder Alkyloxy ausgewählt ist;
ein *N-*Oxid davon, ein pharmazeutisch unbedenkliches Salz davon oder ein Solvat davon.

2. Verbindung nach Anspruch 1, worin
R³ für Alkyl, Arylalkyl, Aryl-O-alkyl, Arylalkyl- O-alkyl, Aryl, Het, Het-Alkyl, Het-O-Alkyl, Het-Alkyl-O-alkyl oder 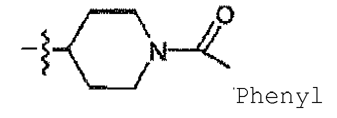 steht;
R⁴ und R⁵ jeweils unabhängig voneinander für Wasserstoff; Alkyl; Alkyloxyalkyl; Arylalkyl; Het-Alkyl; Mono- oder Dialkylaminoalkyl; Het; Aryl oder -C(=NH)-NH₂ stehen; oder
R⁴ und R⁵ gemeinsam mit dem Stickstoffatom, an das sie gebunden sind, einen Rest aus der Gruppe bestehend aus Pyrrolidino, Piperidino, Piperazino, Morpholino, 4-Thiomorpholino, 2,3- Dihydroisoindol-1-yl, Thiazolidin-3-yl, 1,2,3,6-Tetrahydropyridyl, Hexahydro-1*H-* azepinyl, Hexahydro-1*H-*1,4-diazepinyl, Hexahydro-1,4-oxazepinyl, 1,2,3,4- Tetrahydroisochinolin-2-yl, 2,5-Diazabicyclo- [2.2.1]heptyl, Pyrrolinyl, Pyrrolyl, Imidazolidinyl, Pyrazolidinyl, 2-Imidazolinyl, 2-Pyrazolinyl, Imidazolyl, Pyrazolyl, Triazolyl, Pyridinyl, Pyridazinyl, Pyrimidinyl, Pyrazinyl und Triazinyl bilden, wobei jeder Rest gegebenenfalls durch 1, 2, 3 oder 4 Substituenten substituiert ist, wobei jeder Substituent unabhängig unter Alkyl, Halogen- alkyl, Alkylcarbonyl, Halogen, Arylalkyl, Hydroxy, Alkyloxy, Amino, Mono- oder Dialkyl- amino, Alkylthio, Alkylthioalkyl, Aryl, Pyridyl, Pyrimidinyl, Piperidinyl oder Pyrrolidinyl, das gegebenenfalls durch Aryl- alkyl substituiert ist, ausgewählt ist;
Aryl für einen Homocyclus steht, der unter Phenyl, Naphthyl, Acenaphthyl oder Tetrahydronaphthyl ausgewählt ist, wobei jeder dieser Cyclen gegebenenfalls durch 1, 2 oder 3 Substituenten substituiert ist, wobei jeder Substituent unabhängig unter Hydroxy, Halogen, Cyano, Nitro, Amino, Mono- oder Dialkylamino, Alkyl, Halogenalkyl, Alkyloxy, Halogenalkyloxy, Carboxyl, Alkyloxycarbonyl, Aminocarbonyl, Morpholinyl oder Mono- oder Dialkylamino- carbonyl ausgewählt ist.

3. Verbindung nach Anspruch 1 oder 2, worin Alkyl für C₁₋₆-Alkyl steht.

4. Verbindung nach einem der vorhergehenden Ansprüche, worin R¹ für Halogen, Het oder Aryl steht.

5. Verbindung nach Anspruch 4, worin R¹ für Halogen steht.

6. Verbindung nach einem der vorhergehenden Ansprüche, worin p gleich 1 ist.

7. Verbindung nach einem der vorhergehenden Ansprüche, worin R² für C₁₋₆-Alkyloxy steht.

8. Verbindung nach einem der vorhergehenden Ansprüche, worin R³ für Aryl steht.

9. Verbindung nach einem der vorhergehenden Ansprüche, worin q für 2, 3 oder 4 steht.

10. Verbindung nach einem der vorhergehenden Ansprüche, worin R⁴ und R⁵ für C₁₋₆-Alkyl stehen.

11. Verbindung nach einem der vorhergehenden Ansprüche, worin R⁷ für Wasserstoff steht.

12. Verbindung nach einem der vorhergehenden Ansprüche, bei der es sich um eine Verbindung der Formel (Ia) handelt.

13. Verbindung nach Anspruch 12, worin R¹ für Halogen steht; R² für C₁₋₆-Alkyloxy steht; R³ für Aryl steht; R⁴ und R⁵ für C₁₋₆-Alkyl stehen; R⁷ für Wasserstoff steht; q für 2, 3 oder 4 steht und p für 1 steht.

14. Verbindung nach einem der vorhergehenden Ansprüche zur Verwendung als Arzneimittel.

15. Verbindung nach einem der Ansprüche 1 bis 13 zur Verwendung als Arzneimittel für die Behandlung einer bakteriellen Infektion einschließlich einer mykobakteriellen Infektion.

16. Pharmazeutische Zusammensetzung, enthaltend einen pharmazeutisch unbedenklichen Träger und als Wirkstoff eine therapeutisch wirksame Menge einer Verbindung gemäß einem der Ansprüche 1 bis 13.

17. Verbindung nach Anspruch 15, wobei es sich bei der bakteriellen Infektion um eine Infektion mit einem grampositiven Bakterium handelt.

18. Verbindung nach Anspruch 15, wobei es sich bei dem grampositiven Bakterium um Streptococcus pneumoniae oder Staphylococcus aureus handelt.

19. Kombination aus (a) einer Verbindung nach einem der Ansprüche 1 bis 13 und (b) einem oder mehreren anderen antibakteriellen Mitteln.

20. Produkt, enthaltend (a) eine Verbindung nach einem der Ansprüche 1 bis 13 und (b) ein oder mehrere andere antibakterielle Mittel, als Kombinationspräparat für die gleichzeitige, getrennte oder aufeinanderfolgende Verwendung bei der Behandlung einer bakteriellen Infektion.

21. Verbindung nach Anspruch 18, wobei es sich bei dem grampositiven Bakterium um methicillinresistenten *Staphylococcus aureus* handelt.

## Revendications

1. Composé de formule (Ia) ou (Ib) 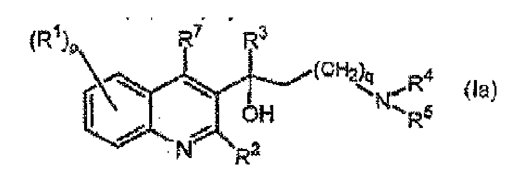 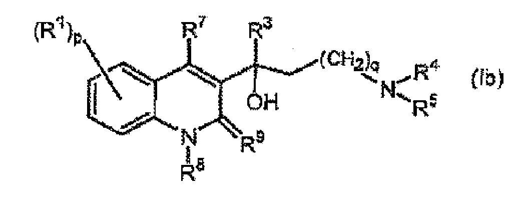 comprenant une forme isomère stéréochimique quelconque de celui-ci, dans lequel
p est un entier égal à 1, 2, 3 ou 4;
q est un entier égal à zéro, 1, 2, 3 ou 4;
R¹ est un hydrogène, un cyano, un formyle, un carboxyle, un halogéno, un alkyle, un alcényle en C₂₋₆, un alcynyle en C₂₋₆, un halogénoalkyle, un hydroxy, un alkyloxy, un alkylthio, un alkylthioalkyle, -C=N-OR¹¹, un amino, un mono- ou di(alkyl)amino, un aminoalkyle, un mono- ou di(alkyl)aminoalkyle, un alkylcarbonylaminoalkyle, un aminocarbonyle, un mono- ou di(alkyl)aminocarbonyle, un arylalkyle, un arylcarbonyle, R^{5a}R^{4a}Nalkyle, un di (aryl) alkyle, un aryle, R^{5a}R^{4a}N-, R^{5a}R^{4a}N-C (=O) -, ou Het ;
R² est un hydrogène, un alkyloxy, un aryle, un aryloxy, un hydroxy, un mercapto, un alkyloxyalkyloxy, un alkylthio, un mono- ou di(alkyl)amino, un pyrrolidino
ou un radical de formule 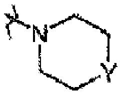 dans lequel Y est CH₂, O, S, NH ou *N-*alkyle ;
R³ est un alkyle, un arylalkyle, un aryl-O-alkyle, un ryl-alkyl-O-alkyle, un aryle, un aryl-aryle, Het, Het-alkyle, Het-O-alkyle, Het-alkyl-O-alkyle ou 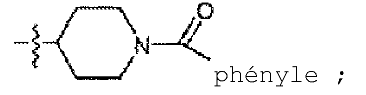 R⁴ et R⁵ chacun indépendamment est un hydrogène ; un alkyle ; un alkylxoyalkyle; un arylalkyle; Het-alkyle ; un mono- ou dialkylaminoalkyle; un bicyclo[2.2.1]heptyle; Het; un aryle; ou -C(=NH)-NH₂; ou
R⁴ et R⁵ conjointement avec l'atome d'azote auquel ils sont liés forment un radical choisi dans le groupe constitué d'un pyrrolidino, un pipéridino, un pipérazino, un morpholino, un 4-thiomorpholino, un 1,1-dioxyde-thiomorpholinyle, un azétidinyle, un 2,3-dihydroisoindol-1-yle, un thiazolidin-3-yle, un 1,2,3,6-tétrahydropyridyle, un hexahydro-1*H-*azépinyle, un hexahydro-1*H-*1,4-diazépinyle, un hexahydro-1,4-oxazépinyle, un 1,2,3,4-tétrahydroisoquinoléin-2-yle, un 2,5-diazabicyclo[2.2.1]heptyle, un pyrrolinyle, un pyrrolyle, un imidazolidinyle, un pyrazolidinyle, un 2-imidazolinyle, un 2-pyrazolinyle, un imidazolyle, un pyrazolyle, un triazolyle, un pyridinyle, un pyridazinyle, un pyrimidinyle, un pyrazinyle et un triazinyle, chaque radical étant facultativement substitué par 1, 2, 3 ou 4 substituants, chaque substituant étant indépendamment choisi parmi un alkyle, un halogénoalkyle, un alkylcarbonyle, un halogéno, un arylalkyle, un hydroxy, un alkyloxy, un amino, un mono- ou dialkylamino, un aminoalkyle, un mono- ou dialkylaminoalkyle, un alkylthio, un alkylthioalkyle, un aryle, un pyridyle, un pyrimidinyle, un pipéridinyle facultativement substitué par un alkyle ou un pyrrolidinyle facultativement substitué par un arylalkyle ;
R^{4a} et R^{5a} conjointement avec l'atome d'azote auquel ils sont liés forment un radical choisi dans le groupe constitué d'un pyrrolidino, un pipéridino, un pipérazino, un morpholino, un 4-thiomorpholino, un 2,3-dihydroisoindol-1-yle, un thiazolidin-3-yle, un 1,2,3,6-tétrahydropyridyle, un hexahydro-1H-azépinyle, un hexahydro-1H-1,4-diazépinyle, un hexahydro-1,4-oxazépinyle, un 1,2,3,4-tétrahydroisoquinoléin-2-yle, un pyrrolinyle, un pyrrolyle, un imidazolidinyle, un pyrazolidinyle, un 2-imidazolinyle, un 2-pyrazolinyle, un imidazolyle, un pyrazolyle, un triazolyle, un pyridinyle, un pyridazinyle, un pyrimidinyle, un pyrazinyle et un triazinyle, chaque radical étant facultativement substitué par 1, 2, 3 ou 4 substituants, chaque substituant étant indépendamment choisi parmi un alkyle, un halogénoalkyle, un halogéno, un arylalkyle, un hydroxy, un alkyloxy, un amino, un mono- ou dialkylamino, un alkylthio, un alkylthioalkyle, un aryle, un pyridyle ou un pyrimidinyle ;
R⁷ est un hydrogène, un halogéno, un alkyle, un aryle ou Het ;
R⁸ est un hydrogène ou un alkyle ;
R⁹ est un oxo ; ou
R⁸ et R⁹ forment conjointement le radical -CH=CH-N=;
R¹¹ est un hydrogène ou un alkyle ;
un aryle est un homocycle choisi parmi un phényle, un naphtyle, un acénaphtyle ou un tétrahydronaphtyle, chacun étant facultativement substitué par 1, 2 ou 3 substituants, chaque substituant étant indépendamment choisi parmi un hydroxy, un halogéno, un cyano, un nitro, un amino, un mono- ou dialkylamino, un alkyle, un alcényle en C₂₋₆ facultativement substitué par un phényle, un halogénoalkyle, un alkyloxy, un halogénoalkyloxy, un carboxyle, un alkyloxycarbonyle, un aminocarbonyle, un morpholinyle ou un mono- ou dialkylaminocarbonyle ;
Het est un hétérocycle monocyclique choisi parmi un N-phénoxypipéridinyle, un pipéridinyle, un pyrrolyle, un pyrazolyle, un imidazolyle, un furanyle, un thiényle, un oxazolyle, un isoxazolyle, un thiazolyle, un isothiazolyle, un pyridinyle, un pyrimidinyle, un pyrazinyle ou un pyridazinyle ; ou un hétérocycle bicyclique choisi parmi un quinoléinyle, un quinoxalinyle, un indolyle, un benzimidazolyle, un benzoxazolyle, un benzisoxazolyle, un benzothiazolyle, un benzisothiazolyle, un benzofuranyle, un benzothiényle, un 2,3-dihydrobenzo[1,4]dioxinyle ou un benzo[1,3]dioxolyle ; chaque hétérocycle monocyclique et bicyclique étant facultativement substitué par 1, 2 ou 3 substituants, chaque substituant étant indépendamment choisi parmi un halogéno, un hydroxy, un alkyle ou un alkyloxy ;
un N-oxyde de celui-ci, un sel pharmaceutiquement acceptable de celui-ci ou un solvate de celui-ci.

2. Composé selon la revendication 1 dans lequel R³ est un alkyle, un arylalkyle, un aryl-O-alkyle, un aryl-alkyl-O-alkyle, un aryle, Het, Het-alkyle, Het-O-alkyle, Het-alkyl-O-alkyle ou 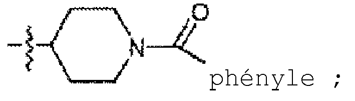 R⁴ et R⁵ chacun indépendamment est un hydrogène ; un alkyle ; un alkylxoyalkyle ; un arylalkyle ; Het-alkyle ; un mono- ou dialkylaminoalkyle ; Het ; un aryle ; ou -C(=NH)-NH2 ; ou
R⁴ et R⁵ conjointement avec l'atome d'azote auquel ils sont liés forment un radical choisi dans le groupe constitué d'un pyrrolidino, un pipéridino, un pipérazino, un morpholino, un 4-thiomorpholino, un 2,3-dihydroisoindol-1-yle, un thiazolidin-3-yle, un 1,2,3,6-tétrahydropyridyle, un hexahydro-1H-azépinyle, un hexahydro-1H-1,4-diazépinyle, un hexahydro-1,4-oxazépinyle, un 1,2,3,4-tétrahydroisoquinoléin-2-yle, un 2,5-diazabicyclo[2.2.1]heptyle, un pyrrolinyle, un pyrrolyle, un imidazolidinyle, un pyrazolidinyle, un 2-imidazolinyle, un 2-pyrazolinyle, un imidazolyle, un pyrazolyle, un triazolyle, un pyridinyle, un pyridazinyle, un pyrimidinyle, un pyrazinyle et un triazinyle, chaque radical étant facultativement substitué par 1, 2, 3 ou 4 substituants, chaque substituant étant indépendamment choisi parmi un alkyle, un halogénoalkyle, un alkylcarbonyle, un halogéno, un arylalkyle, un hydroxy, un alkyloxy, un amino, un mono- ou dialkylamino, un alkylthio, un alkylthioalkyle, un aryle, un pyridyle, un pyrimidinyle, un pipéridinyle ou un pyrrolidinyle facultativement substitué par un arylalkyle ;
un aryle est un homocycle choisi parmi un phényle, un naphtyle, un acénaphtyle ou un tétrahydronaphtyle, chacun étant facultativement substitué par 1, 2 ou 3 substituants, chaque substituant étant indépendamment choisi parmi un hydroxy, un halogéno, un cyano, un nitro, un amino, un mono- ou dialkylamino, un alkyle, un halogénoalkyle, un alkyloxy, un halogénoalkyloxy, un carboxyle, un alkyloxycarbonyle, un aminocarbonyle, un morpholinyle ou un mono- ou dialkylaminocarbonyle.

3. Composé selon la revendication 1 ou 2 dans lequel un alkyle représente un alkyle en C₁₋₆.

4. Composé selon l'une quelconque des revendications précédentes dans lequel R¹ est un halogéno, Het ou un aryle.

5. Composé selon la revendication 4 dans lequel R¹ est un halogéno.

6. Composé selon l'une quelconque des revendications précédentes dans lequel p est égal à 1.

7. Composé selon l'une quelconque des revendications précédentes dans lequel R² est un alkyloxy en C₁₋₆.

8. Composé selon l'une quelconque des revendications précédentes dans lequel R³ est un aryle.

9. Composé selon l'une quelconque des revendications précédentes dans lequel q est égal à 2, 3 ou 4.

10. Composé selon l'une quelconque des revendications précédentes dans lequel R⁴ et R⁵ représentent un alkyle en C₁₋₆.

11. Composé selon l'une quelconque des revendications précédentes dans lequel R⁷ est un hydrogène.

12. Composé selon l'une quelconque des revendications précédentes dans lequel le composé est un composé de formule (Ia).

13. Composé selon la revendication 12 dans lequel R¹ est un halogéno ; R² est un alkyloxy en C₁₋₆ ; R³ est un aryle ; R⁴ et R⁵ sont un alkyle en C₁₋₆ ; R⁷ est un hydrogène ; q est 2, 3 ou 4 ; et p est 1.

14. Composé selon l'une quelconque des revendications précédentes pour utilisation en tant que médicament.

15. Composé selon l'une quelconque des revendications 1 à 13 pour utilisation en tant que médicament pour le traitement d'une infection bactérienne comprenant une infection mycobactérienne.

16. Composition pharmaceutique comprenant un véhicule pharmaceutiquement acceptable et, en tant que substance active, une quantité thérapeutiquement efficace d'un composé tel que défini dans l'une quelconque des revendications 1 à 13.

17. Composé selon la revendication 15 où l'infection bactérienne est une infection par une bactérie Gram positif.

18. Composé selon la revendication 15 où la bactérie Gram positif est *Streptococcus pneumoniae* ou *Staphylococcus aureus.*

19. Combinaison de (a) un composé selon l'une quelconque des revendications 1 à 13, et (b) un ou plusieurs autres agents antibactériens.

20. Produit contenant (a) un composé selon l'une quelconque des revendications 1 à 13, et (b) un ou plusieurs autres agents antibactériens, sous la forme d'une préparation combinée pour utilisation simultanée, séparée ou séquentielle dans le traitement d'une infection bactérienne.

21. Composé selon la revendication 18 où la bactérie Gram positif est *Staphylococcus aureus* résistant à la méthicilline.
